# EUROPEAN PATENT SPECIFICATION

(11) **EP 4 009 899 B1**
(45) Date of publication and mention of the grant of the patent: **26.07.2023**
(21) Application number: 20764765.2
(22) Date of filing: 30.07.2020
(51) Int. Cl.: A61C 1/00, A61B 17/00, A61C 17/16

(54) **HANDPIECE ASSEMBLY FOR MEDICAL DEVICE**
HANDSTÜCKANORDNUNG FÜR MEDIZINPRODUKT
ENSEMBLE PIÈCE À MAIN DESTINÉ À UN DISPOSITIF MÉDICAL

(30) Priority: 09.08.2019 IT 201900014565
(43) Date of publication of application: 15.06.2022
(73) Proprietor: Mectron S.p.A., 16042 Carasco GE (IT)
(72) Inventor: MINUTOLI, Saverio, 16042 Carasco, Genova (IT)
(74) Representative: Brunazzi, Stefano
(86) International application number: PCT/IB2020/057193
(87) International publication number: WO 2021/028764

(56) References cited:
- CN-A- 101 807 243
- US-A1- 2008 293 008
- US-A1- 2018 256 287

## Description

### Field of the invention

The present invention relates to a handpiece assembly for a medical device.

In particular, the present invention relates to a handpiece assembly for a medical device comprising a handpiece, adapted to of accommodate by insertion an insert assembly and a handpiece antenna.

For example, the present invention relates to a handpiece assembly for ultrasonic piezoelectric devices suited to recognize inserts, or insert tips, by means of a miniaturized radiofrequency identifier.

Furthermore, the present invention relates to the context of an ultrasonic system which is particularly and advantageously applied in the medical-surgical field (e.g., neuro-spinal, craniofacial, orthopedic, otorhinolaryngological), in dental surgical and non-surgical fields (e.g., oral surgery, implantology, dental hygiene and prophylaxis, etc.) but which is equally usable in the industrial or construction field according to other embodiments.

More precisely, such system can be used in sectors in which it is necessary to perform a removal, abrasion, cutting or drilling of material, e.g., mineralized material.

Hereinafter, application will mean application in the medical-surgical field (e.g. ,neuro-spinal, craniofacial, orthopedic, otorhinolaryngological), dental field, surgical and non-surgical (e.g., in oral surgery, implantology, dental hygiene and prophylaxis, etc.) but it is equally usable in the industrial or constructions field according to other embodiments, sectors in which it is necessary to perform a removal, abrasion, cutting or drilling of material, e.g., mineralized type, such as bone, enamel, dentin, dental calculus, and biofilm.

### Prior art

In the medical or dental field, and specifically in the surgical or implantological field, power ultrasounds are applied in the dissection of hard tissues (bone) and soft tissues, in the cauterization of blood vessels and in the field of dental prophylaxis for the removal of tartar.

Referring to the field of implantology by way of example only, the sites for the insertion of screws or other fixing systems into the bone are prepared by using rotating tools of the aforementioned type, which however have serious limitations both intrasurgery for the operator and post-surgery for the patient.

Just to mention a few, the traditional instruments are problematic when operating on surgical sites in the presence of complex anatomical structures of difficult or limited surgical access, or near delicate anatomical structures, such as nerves and blood vessels.

The large amount of mechanical energy produced by the rotation and the considerable pressure that the operator must apply onto the instrument are responsible for possible damage to non-mineralized structures, for the production of a considerable amount of heat, for losses due to friction, with a consequent overheating of the mineralized tissues, for operator fatigue at the expense of the required intra-surgical precision and control.

The context of the present invention relates to ultrasonic power systems for medical and dental use, e.g. as the use for oral implantology. However, this invention can be equally applied to other fields of the medical and industrial sector.

The operation of most ultrasonic power systems is based on the transmission of longitudinal waves in the application means. Such waves are generated by piezoelectric transducers and transferred into the means through concentrators or waveguides named ultrasonic horns.

However, there are applications in which flexural, torsional or compound vibrations are used. In the dental field, for example, longitudinal vibrations excited in the ultrasound transducers are converted into flexural oscillations through asymmetrically shaped tips or insert tips. The incorporation of one or more curves in the insert tip profile has a dual purpose, i.e., to allow a good access to the inside of the oral cavity and to convert the longitudinal movement of the transducer into a linear flexural vibration close to the operating part of the insert tip.

In ultrasound ablators, the flexural movement of hooked-shaped inserts / insert tips is normally used to remove calcified deposits (tartar) from the teeth. In ultrasound scalpels (such as the "*Piezosurgery device*" made by Mectron S.p.A.) the transversal movement produced in scythe-shaped inserts / insert tips is used to dissect mandibular bones and other mineralized tissues with precision.

There are also ultrasonic ablators which remove tartar through both linear and elliptical oscillations, as described in DE102005044074A1 or in EP2057960B1**.** In these systems, vibratory movements having bidirectional components are generated in the insert tips by means of flexural vibrations of the transducer on orthogonal planes, see in particular EP2057960B1**.** The configurations of these flexural transducers refer to a previously disclosed concept in which transverse oscillation is caused by adjacent piezoelectric volumes inserted radially and axially with opposite polarizations [see Mori, E. et al., "New Bolt Clamped Flexural Mode Ultrasonic High Power Transducer with One-Dimensional Construction", Ultrasonics International 89 Conference Proceedings"; Watanabe, Y. et al., "A Study on a New Flexural-mode Transducer-solid Horn System and its Application to Ultrasonic Plastic Welding", Ultrasonics Vol. 34, 1996, pp. 235-238; Yun, C-H. et al "A High Power Ultrasonic Linear Motor using a Longitudinal and Bending Hybrid Bolt-Clamped Langevin Type Transducer", Jpn. J. Appl. Phys., Vol. 40, 2001, pp. 3773-3776].

In maxillofacial surgery procedures, the ultrasonic oscillations of the insert tips are commonly used to sever bone tissue.

According to the dental implantology protocol, once the first hole of reduced size has been made, it is progressively enlarged using rotary drills of an increasing section until it reaches a diameter compatible with the implant.

The insert tips typically used in ultrasound systems for operations performed in the oral cavity have insufficient oscillatory amplitudes to perform all stages of implant site preparation. Such limitation is inherent in the design of these devices in which the larger the sections of the insert tips, the smaller the amplitude of the produced vibration, the handpiece being equal. This inverse relation between the section and the oscillation of the inserts / insert tips is a limit of applicability of the technology, especially in oral implantology in which drilling holes several millimeters in diameter is necessary.

There is a further problem related to the linear vibration of the insert tips which does not allow the perforation of the mandibular fabric unless a manual swing of the handpiece is applied in combination with it. Such auxiliary movement is certainly difficult to produce by the operator inside the mouth and is in any case not very compatible with the requirements of precision that clinical implantology practice requires today.

Ultrasonic devices capable of dissecting biological tissue by excitation of torsional, or combined torsional and longitudinal ultrasonic vibrations have been known from US7374552B2**,** US6402769B1**,** US2009/236938A1**,** US2011/0278988A1**.** The common feature of these devices is that they all have a single geometric development axis, being basically axial-symmetrical systems. In maxillofacial applications, such as dental implantology, the oscillating insert tips used in the oral cavity have significantly asymmetrical developments relative to the transducer axis. Therefore, in these context, it is not possible to produce torsional or longitudinal and torsional vibrations in the operating parts of the insert tips following the teachings of the mentioned inventions (valid only for systems in which transducer and operating parts are coaxial).

Slipszenko (US2013/0253559A1) devised ultrasonic system configurations in which torsional, flexural, or longitudinal vibrations are alternately produced in ultrasonic scalpels for the treatment of soft tissues with a developmental axis perpendicular to that of the transducer. According to this solution, the transverse vibration of the piezoelectric transducer can be transformed into torsional, flexural, or longitudinal oscillation by incorporating an ultrasonic horn or waveguide mounted eccentrically relative to the transducer axis. For the vibration transmission to take place correctly, the diameter of the back of the horn must be greater than that of the transducer. Although it is possible to generate alternative vibratory families on orthogonal planes, the specific requirements of compactness, ergonomics, and weight of dental and medical devices cannot be achieved by applying Slipszenko's solution. The large size and eccentric mounting of the ultrasonic horn would significantly limit the visibility inside the oral cavity. Furthermore, in Slipszenko's solution, one or more waveguides are inserted between the scalpel and the conversion/ vibratory transmission horn to transmit adequate vibrations. Even reducing the number of these components to a minimum, the overall length of the device would still be incompatible for applications in small, cramped, and delicate spaces, such as inside the oral or maxillofacial or neuro-spinal or skull cavity.

Mishiro (JPH0373207A) suggested an ultrasonic system for the removal of material which could theoretically find applicability in dental applications. The suggested solution is based on an operating principle typical of ultrasonic motors in which the elliptical vibration generated in a joint formed by an ultrasonic transducer coupled to a waveguide produces the rotation of an operating element (tool) kept in contact with the tip of the waveguide. In the configurations illustrated in **JPH0373207A,** the operating element, the axis of symmetry of which can be either perpendicular or parallel to that of the transducer, oscillates ultrasonically allowing the removal of material in addition to rotating. The contact point between the operating element and the waveguide through which the oscillatory movement is transferred is generated by rotation and corresponds to an antinode of the longitudinal and transverse vibrations generated in the joint transducer-waveguide. According to the configurations described in this solution, the operating element is supported by two pads positioned at the same number of stationary nodes produced along the oscillating element. Such solution appears complex in its implementation and unsuitable for applications in which the operating elements (inserts / insert tips) must be used and replaced in succession, as in dental implantology.

Furthermore, in the field of biomedical or medical instruments, the need for continuous improvement of patient safety is strongly felt.

For this reason, it must be guaranteed that inserts / insert tips for biomedical instruments (e.g. insert tips adapted to operate on the teeth of a patient in a medical device for dental use or, for example, on bones in an instrument for surgical use in districts, such as the maxillofacial or neuro-spinal or skull or orthopedic district) always operate correctly and safely, come from reliable manufacturers, are of the right type for the medical device, have not been overused, and so on.

For this purpose, it is very important to have inserts /insert tips equipped with an identifier, capable of providing information about the insert tip and its operation to a control unit of the medical device.

With this regard, insert tips equipped with RFID identifiers are known, capable of communicating wireless with the remaining part of the medical device.

Solutions of this type are, for example, described in WO2006082340**,** US2011087605A1**,** US2015147718A1**,** US2009047624A1**,** US2008044790A1**,** US2009065565A1**,** US2015150647A1, US2008293008.

However, because of the particular characteristics of the inserts / insert tips used in this field, above all the very small size and the presence of a metal element which constitutes the body of the insert tip, the communication performance which is provided by the known solutions is unsatisfactory relative to needs or is not found in applications available to users today.

More specifically, the miniature antennas, arranged in the insert tip to guarantee wireless communication of the RFID identifier with the rest of the medical device, i.e., for the excitation of the RFID identifier, act like inductances.

Such inductances, however, are disturbed by the presence of the metal of the body of the insert tip, and further by possible liquids (e.g., physiological solutions which are saline by their nature) which may be present in the insert tip, which act as antagonists of the electromagnetic fields, because they tend to absorb the electro-magnetic fields and, in the case of metals, to re-emit the electro-magnetic fields symmetrically causing their cancellation in the boundary zone of the metal.

In brief, the overall result of such phenomena is a strong attenuation, or even cancellation, of signals carried by electromagnetic fields in the vicinity of the insert tip antenna, which worsens the performance of the communication between the RFID identifier and the rest of the medical device or even prevents such communications from taking place.
This situation effectively frustrates the advantages of the RFID identifier.

A further drawback of the aforesaid known solutions of inserts / insert tips with RFID identifiers consists of the difficulty in designing insert tip antennas operating at the frequencies required by UHF RFID communication, according to the different standards provided in the various countries for this type of communication.
In summary, the need for inserts/insert tips for medical instruments equipped with identifiers capable of supporting effective and reliable communication with the remaining parts of the medical device remains unsatisfied.

Furthermore, it must be considered that the insert tips must be interchangeably connected to ultrasonic generators and, to be used repeatedly, they must be able to be separated from the handpiece and placed in an autoclave, leading to repeated and very stressful treatment cycles for any RFID identifiers connected to them.

Therefore, the need is still strongly felt for inserts/ insert tip identification solutions which are not only small in size, and thus able to adapt to small distal portions of the handpiece, i.e., adapted to tight operating or intervention fields, but at the same time are very resistant to the stresses imposed by autoclaves or similar treatment cycles for sterilizing them.

The previously mentioned need to put the RFID identifier of the insert tip into wireless communication with the rest of the medical device places additional requirements on the medical device handpiece. Such handpiece must be equipped with a radiofrequency antenna, which must be appropriately powered.

The reading antenna positioned inside the handpiece must meet very stringent dimensional and operational requirements. The dimensions of the antenna must be miniaturized so that the antenna can be contained in the handpiece. This suggests using ring or loop antennas.

However, small ring or loop antennas suffer from several problems. One of the main problems arises from the fact that the currents circulating in the antenna ring, depending on the working frequency and geometric dimensions, tend to have so-called voids, i.e., points in which the current undergoes a phase inversion. Such phenomenon means that the antenna emission is zero in some points, because the current is zero, and also causes other low emission points, in which the phase current is opposite to the primary one. Ultimately, this significantly worsens the transmission performance of the antenna.

A further fundamental need to minimize energy loss and generation of line reflections due to environmental electromagnetic spurious events derives from the need to guarantee the adaptation of antenna impedance in the UHF frequency range to the generator in the medical device, which is not easy to achieve in this scope of application due to the small size of the antenna and available space.

Finally, the need arises to transmit the radiofrequency signals inside the handpiece intended to supply the handpiece antenna, under impedance adaptation conditions, and in absence of radiofrequency connectors.

Therefore, the further need for effective radiofrequency signal transceiver solutions in the handpiece to allow effective wireless communication with the insert tip radiofrequency identifier is still strongly felt and currently not satisfied.

### Solution

The object of the present invention is to provide a handpiece assembly for a medical device which allows to solve the drawbacks described above with reference to the prior art and to respond to the aforesaid needs particularly felt in the considered technical sector.

Such object is achieved by means of a handpiece assembly according to claim 1.

Further embodiments of such handpiece assembly are defined in claims 2-13.

A further purpose of the present invention is to provide a medical device comprising the aforesaid handpiece assembly.

Such object is achieved by a medical device according to claim 14.

A further embodiment of such device is defined in claim 15.

Some of the main advantages which derive from this invention are the following.

The position, thickness and type of materials allow to minimize the increase in diameter of the insert tip, without obstructing the clinician's vision of the surgical site, guaranteeing and maintaining the patient's safety.

The small dimensions and light weight of the materials and RFID chip, i.e., of the materials added to the metal of the insert tip, less than 15 grams, do not significantly affect the mechanical properties of the ultrasonic transducer when it is mechanically coupled to the insert tip, thus leaving the working parameters, such as ultrasonic frequency and amplitude and power of the device, unchanged, always guaranteeing the maximum and safe performance of the medical device.

The position, small dimensions and weight of the materials and RFID chip on the insert tip prevent degradation and ensure that the insert tip maintains its lifetime, efficiency, and effectiveness.

The position and the small increase in insert tip diameter do not affect the simplicity of mechanical coupling between insert tip and transducer by means of a torque wrench or instrument based on the dynamometric method.

The type of RFID system materials on the insert tip is suited to the temperatures used in the sterilization processes, such as autoclaves and/or instrument washers.

The type of RFID coating materials on the insert tip which interfaces with the patient is biocompatible.

The reduced number of layers of the RFID system on the insert tip guarantees the simplicity of the production process, providing a system that is always highly efficient.

The reduced thickness of the RFID system and the consequent minimal increase of the diameter of the insert tip guarantees an adequate concentric area free from the components surrounding the handpiece, guaranteeing that production process tolerances or flexure caused by excessive pressures imposed by the clinician during the operations can put the mechanical parts of the transducer into contact with the insert tip causing variations in the working parameters of the device.

Identification of the insert tip by means of the unique, non-writable code of the RFID insert tip chip.

Identification of the handpiece or handpiece antenna by means of the unique, non-writable code of the RFID handpiece chip.

In particular with regards to the patient's safety, the unique RFID identification, in conjunction with the medical device, allows:
a. secure traceability of the insert tip or handpiece, throughout its life cycle
b. indication to the clinician that the insert tip and handpiece are adequate and compatible
c. indication to the clinician that the selected insert tip is adequate and suitable for the selected intervention type
d. indication to the clinician of the performance of the insert tip, having evaluated the chronology of the current and past manners and times of use
e. instructions to the clinician on appropriate settings of the medical device concerning the insert tip in use
f. indications of clinical protocols and the respective steps of operation
g. predictive maintenance
h. scheduled maintenance
i. sharing data with the manufacturer for statistics and continuous improvement of products and their conditions of use
j. sharing data with the manufacturer for updating medical devices and possible integrations of new products or usage models for greater efficiency and effectiveness of the medical system with insert tip
k. sharing data with universities, training centers, etc.
l. writing medical records and specifically history of the insert tips used for patient treatments.

Easy cleaning and sterilization of all RFID systems, both of the insert tip and of the distal antenna handpiece position and the handpiece and cord joining the medical device to the handpiece, according to the usual clinical protocols.

In some implementations, the possibility of extracting the antenna handpiece for maintenance as well as for cleaning and sterilization.

The data transmission channel of the RFID system shares, where present, the connections for the illumination of the handpiece cone in the distal position, with the advantage of not increasing the possible anomalies due to more connections and wires, and maintaining the flexibility of the cord which joins the medical device to the handpiece.

The present invention, therefore, provides a universal solution applicable both in dental surgery and prophylaxis or maxillofacial surgery or orthopedics or neuro-spinal or otorhinolaryngological or craniofacial surgery, and in other fields in medical and industrial fields.

### Figures

Further features and advantages of the handpiece for medical device according to the invention will be apparent from the following description of preferred embodiments, given by way of indicative, non-limiting examples, with reference to the accompanying figures, in which:
- figure 1 shows a diagrammatic assembly view of a medical device comprising an ultrasonic system with excitation handpiece and an interchangeable insert assembly, e.g., for dental or microsurgical use;

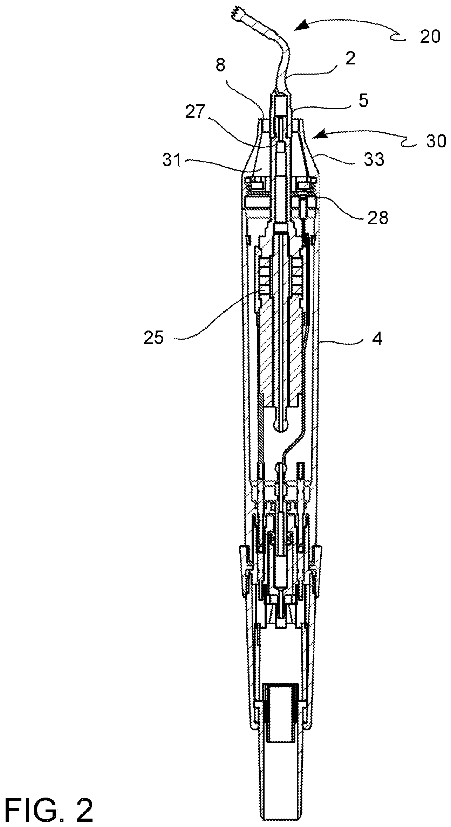

- figure 2 illustrates a section view taken along line II-II in figure 1 of the part of the handpiece assembly and insert assembly in figure 1, in which the components of the handpiece, such as a transducer, e.g., a piezoelectric transducer, are highlighted;

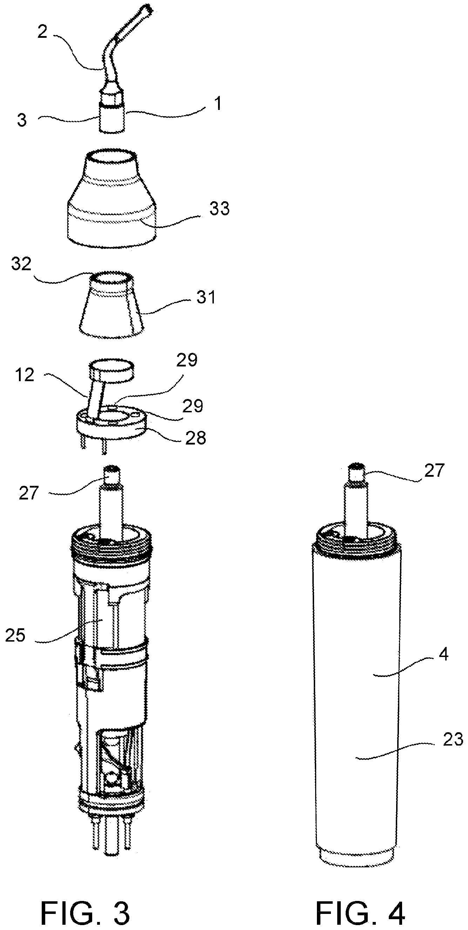

- figure 3 illustrates an axonometric view with parts separated of some components of the handpiece assembly and insert assembly of figure 1 in which the insert assembly, the distal handpiece portion cover, the light guide with light concentrators, the handpiece antenna supported to the handpiece antenna connection element and the inner handpiece components, such as the piezoelectric transducer, are shown;
- figure 4 is an axonometric view of only the central portion of the handpiece without the distal portion cover and the handpiece antenna highlighting the transducer shaft insert connection tang for the interchangeable connection of insert assemblies;

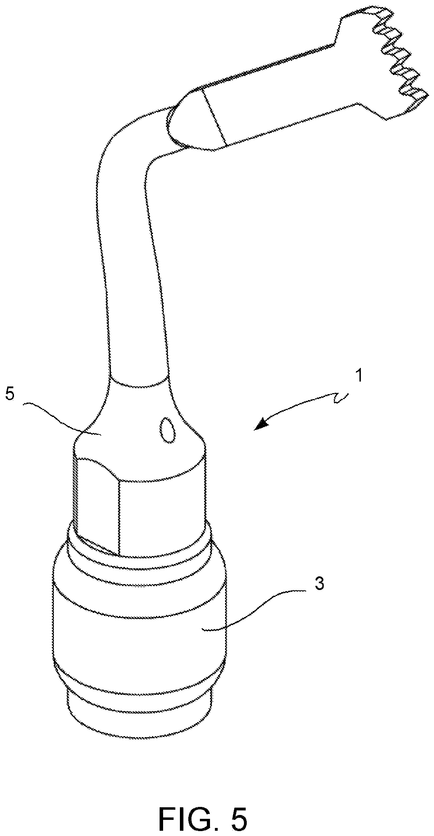

- figure 5 is an axonometric view of an insert assembly;

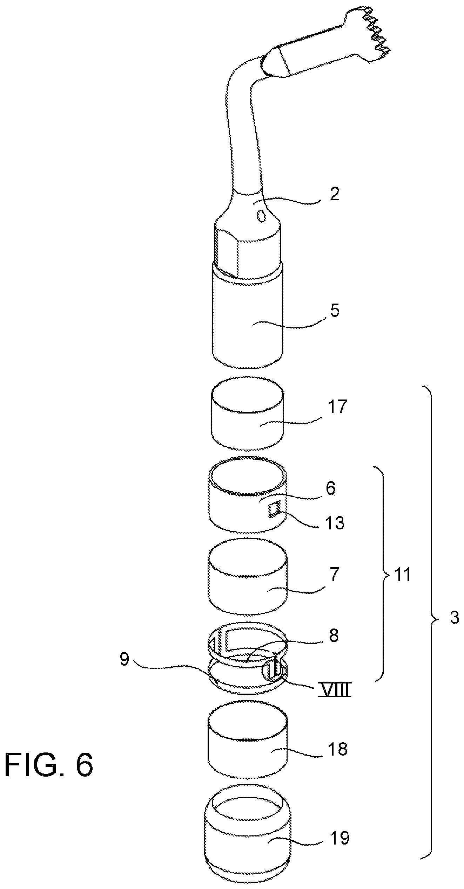

- figure 6 illustrates an axonometric view of the insert tip of figure 5 with parts separated in which the different layers of a radiofrequency identifier are highlighted;

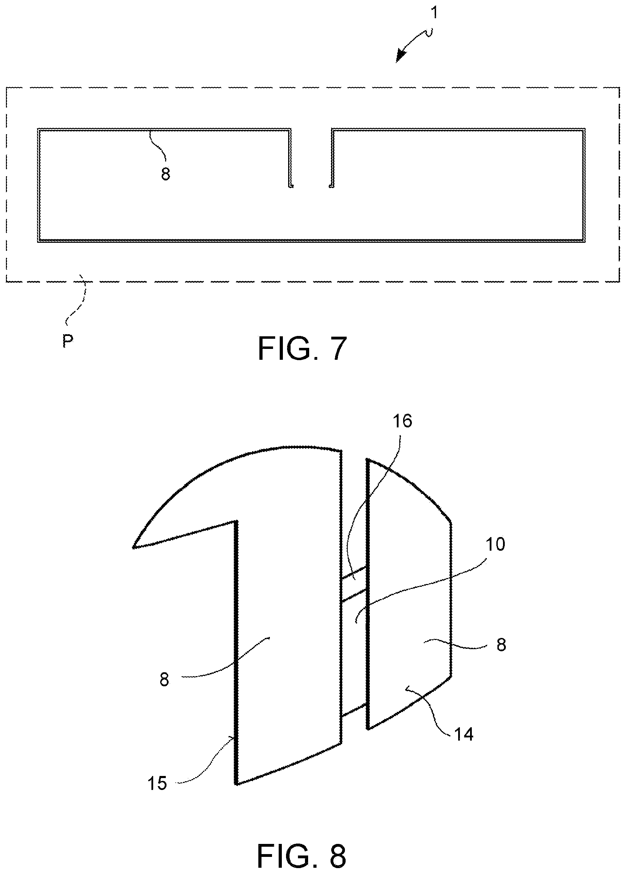

- figure 7 shows an enlarged plan view of the insert antenna in its extension of its planar profile suited to be wound about and surround or partially surround the insert tip foot of an insert tip;
- figure 8 is an axonometric view of a detail of an insert tip antenna to which an identification chip is connected, so as to arrange said identification chip with its entire body protruding from only one side of the extension plane of said insert antenna;

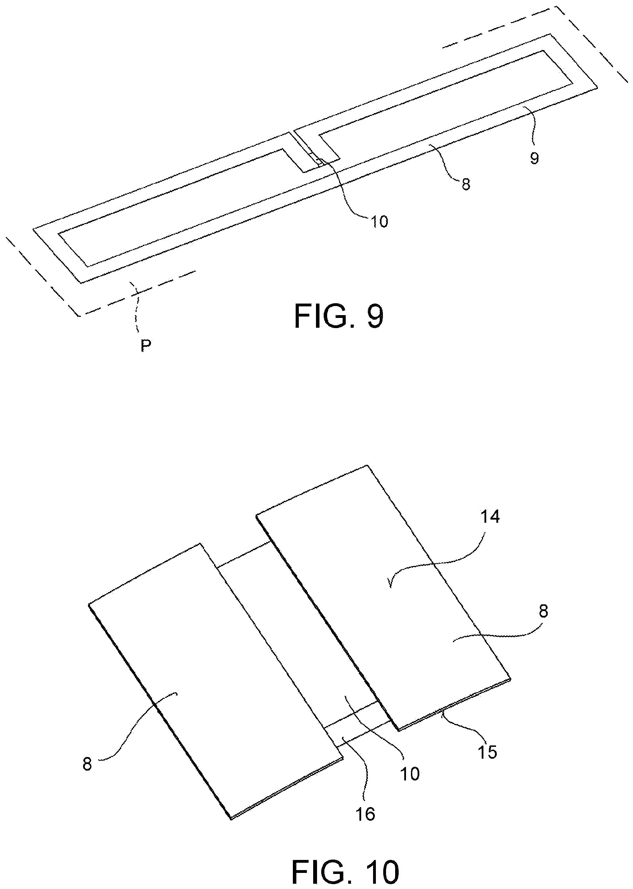

- figure 9 is an axonometric view of a further embodiment of an insert antenna connected to an identification chip;
- figure 10 is an axonometric view of a detail of the insert antenna in figure 9 in the detail in which the identification chip is connected, so as to arrange said identification chip protruding with its entire body from only one side of the extension plane of said insert antenna;

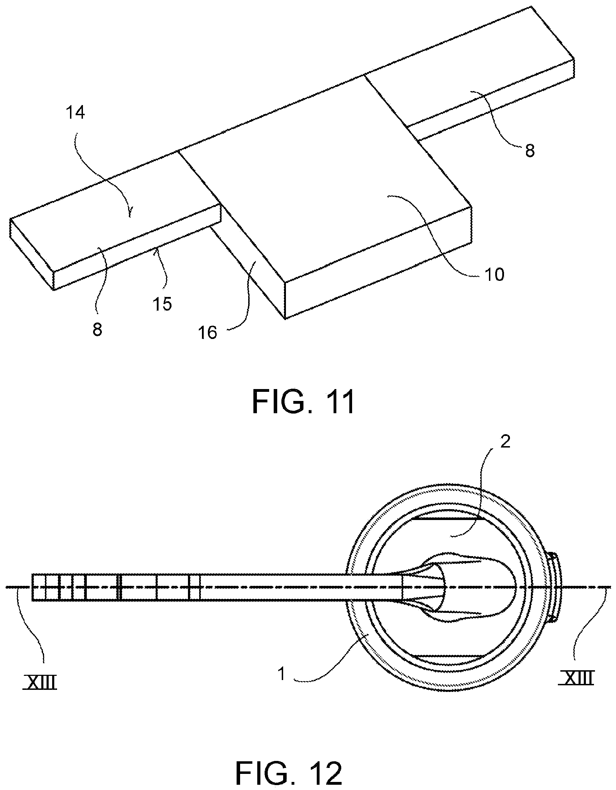

- figure 11 is an axonometric view of a detail of an insert antenna to which an identification chip is connected, so as to place said identification chip with its entire body on the same level as said insert antenna and partially beyond or protruding beyond only one side of the extension plane of said insert antenna;
- figure 12 illustrates a top view of an insert assembly;

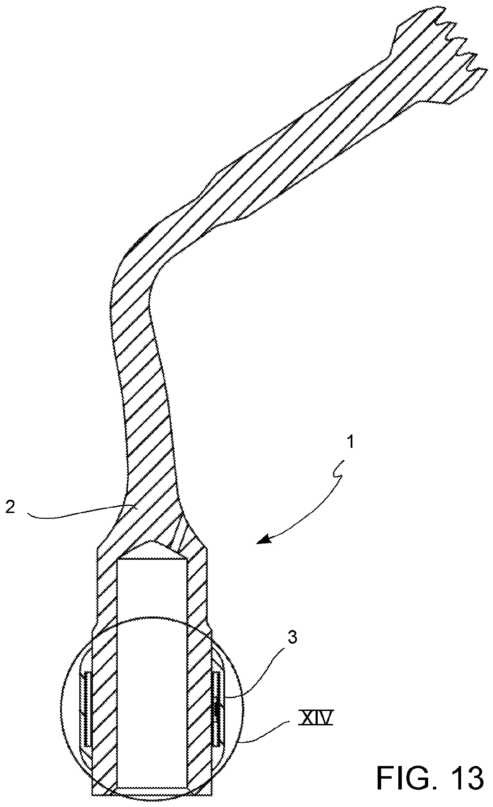

- figure 13 illustrates a section taken along the line XIII-XIII in figure 12 of the insert of figure 12 in which the radiofrequency identifier is highlighted;

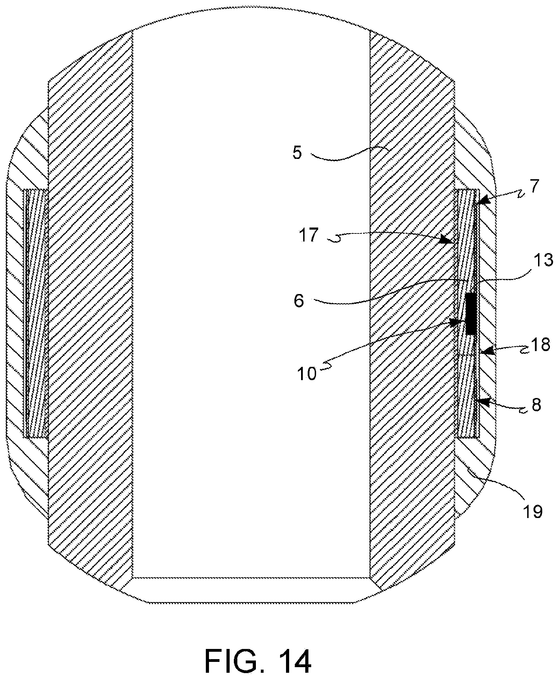

- figure 14 illustrates the enlargement indicated by XIV in figure 13 in which the layers of the radiofrequency identifier are highlighted;

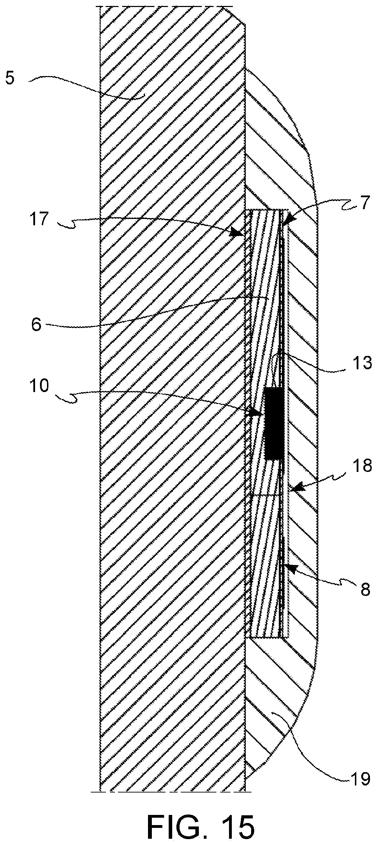

- figure 15 illustrates a section view of the enlargement in figure 14;

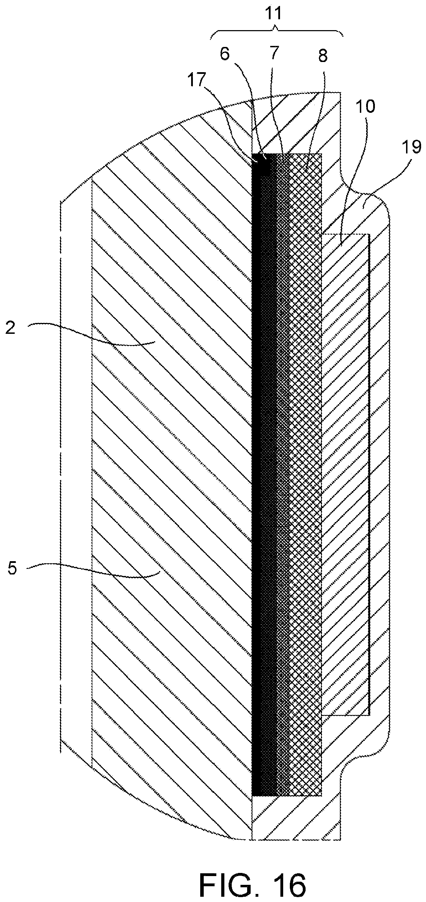

- figure 16 is a detail of a cross-section of an insert assembly according to a further embodiment;

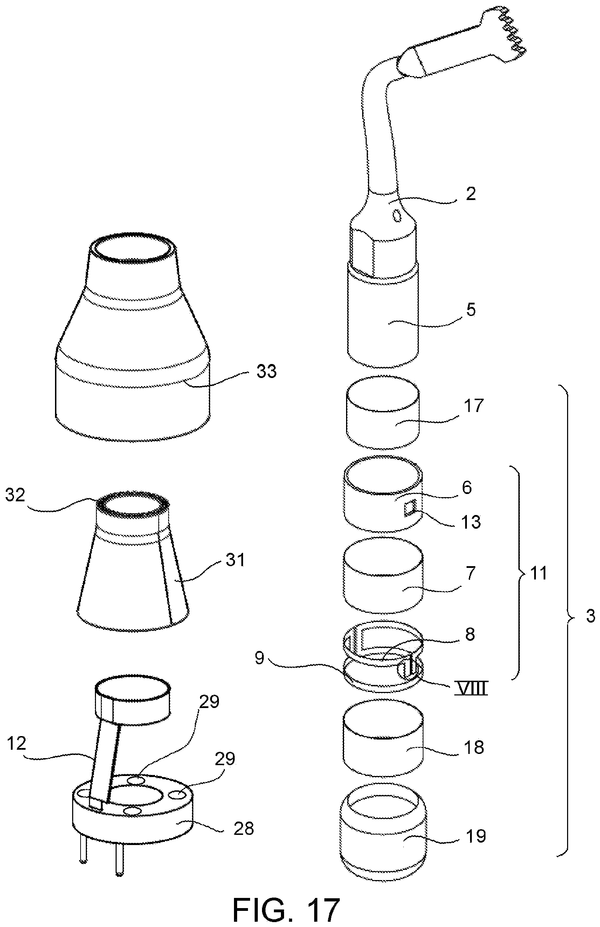

- figure 17 is an axonometric view with parts separated of a handpiece antenna together with the components of the handpiece distal portion and an insert assembly comprising the various layers of a radiofrequency identifier associated with an insert tip;
- figure 18 illustrates an example of an equivalent electrical circuit in serial representation of the assembly formed by the RFID identification chip and the insert antenna;
- figure 19 illustrates a parallel representation of another example of an equivalent electrical circuit of the assembly formed by the RFID identification chip and the insert antenna, also including a connection model;
- figure 20 is a block chart of an embodiment of the insert antenna and the identification chip;
- figure 21 illustrates a block chart of a medical device handpiece portion containing a handpiece antenna;
- figure 22 illustrates a simplified block chart of the RF signal distribution mode in the handpiece;
- figure 23 illustrates a block chart of a coaxial cable connection to connect the handpiece and a medical system control unit;
- figure 24 illustrates an equivalent circuit of an embodiment of a handpiece antenna.

### Description of some preferred embodiments

The term "miniaturized" means a device or component having a size between 50 micrometers and 800 micrometers, preferably between 100 micrometers and 600 micrometers.

The term "medical device" means an electromechanical device in which a piezoelectric handpiece actuates the mechanical movement of ultrasonic frequency insert tips. These devices can be applied in several fields, of which the following are listed as examples only:
- medical: in particular, surgery in the neuro-spinal, cranio-maxillofacial, orthopedic, otorhinolaryngological, pediatric disciplines;
- dental, and in particular, surgery, dentistry in general, hygiene and prophylaxis (in particular, the removal of dental calculi, plaque and biofilm).

The function carried out by the device on bone or on tooth means, for example:
- cutting;
- perforating;
- removing;
- eroding.

The function performed by the device on calculi/plate/biofilm means, for example:
- removing;
- disintegrating.

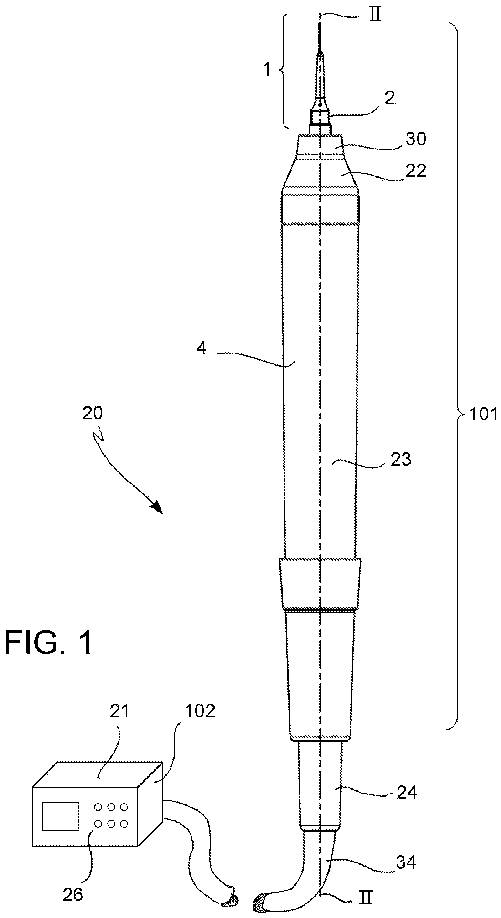

In figure 1, reference numeral 101 identifies an ultrasonic system 101 as a whole comprising generator means 102 or control element 21 operationally connected to a transducer 25 which generates ultrasonic micro-vibrations, which generate vibrations in a connected insert tip 2.

By way of example only, an ultrasonic system 101 is a surgical or prophylactic instrument, e.g., a dental or medical instrument. According to other embodiments, the present ultrasonic system 101 is an industrial instrument.

An insert assembly 1 with radiofrequency identifier 3 is described hereinafter. Such insert assembly 1 is adapted to be inserted into a handpiece 4 of a medical device.

The insert assembly 1 comprises, according to a general embodiment, an insert 2, a ferromagnetic layer 6, a dielectric layer 7, an insert antenna 8, and an identification chip 10.

The insert (insert tip) 2 is designed to interact with a part of the patient's body and comprises at least one insert metal tang 5.

The ferromagnetic layer 6 is arranged in contact with, or on the part of, the aforementioned insert metal tang 5 of the insert 2.

According to an embodiment, said ferromagnetic layer 6 is glued to said insert metal tang 5 of the insert 2. According to a different embodiment, said ferromagnetic layer 6 is applied to said metal tang 5 by interposing a double-sided adhesive.

The ferromagnetic layer 6 comprises ferromagnetic material.

The dielectric layer 7 is arranged in contact with the aforesaid ferromagnetic layer 6.

The insert tip antenna 8 is arranged in contact with the aforesaid dielectric layer 7 and comprises an insert antenna metal element 9, which extends along a predefined, essentially planar profile P.

Such insert antenna 8 is configured to receive and transmit electromagnetic fields, either modulated or non-modulated, within a given frequency range.

An identification chip 10 is operationally connected to the aforesaid insert tip antenna 8 and is configured to transmit, when activated, information related to the insert assembly 1.

The aforesaid ferromagnetic layer 6 is adapted to reduce or cancel attenuation and/or distortion phenomena of the electromagnetic field caused by field parasite effects in the vicinity of the insert antenna 8 due to the interaction of a transmitted/received electromagnetic field with metal parts of the insert metal tang 5 of the insert 2 and/or with liquids present in the insert 2 and/or with the insert antenna metal element 9.

The aforesaid ferromagnetic layer 6, dielectric layer 7, and insert antenna 8 form a transceiver device 11, adapted to wirelessly connecting the identification chip 10 to a handpiece antenna 12 comprised in the handpiece 4 into which insert assembly 1 is inserted.

According to a further embodiment, an insert assembly 1 comprises an insert tip 2 and a radiofrequency identifier 3.

Said insert assembly 1 is adapted to be inserted into a medical device handpiece 4.

Said insert assembly 1 comprises:
- the insert tip 2 adapted to interact with a part of the patient's body, in which the insert tip 2 comprises an insert metal tang 5;
- the radiofrequency identifier 3.

Said radiofrequency identifier comprises a ferromagnetic layer 6, arranged on the side of said insert metal tang 5 of the insert tip 2, in other words directly in contact considering a layer of glue or double-sided adhesive interposed between said ferromagnetic layer 6 and said insert metal tang 5.

Said ferromagnetic layer 6 comprises ferromagnetic material. In particular, said ferromagnetic layer 6 is adapted to reduce or cancel phenomena of attenuation and/or distortion of the electromagnetic field caused by field parasitic effects in the vicinity of an insert antenna 8 due to the interaction of a transmitted/received electromagnetic field with metal parts of the insert metal tang 5; and/or with liquids present in the insert tip and/or with the insert antenna metal element 9.

Said radiofrequency identifier further comprises a dielectric layer 7, arranged in contact with said ferromagnetic layer 6.

Said radiofrequency identifier further comprises said insert antenna 8 arranged in contact with the said dielectric layer 7 and comprising an insert antenna metal element 9, which extends along a predefined, substantially planar profile P.

Said insert antenna 8 is configured to receive and transmit electromagnetic fields within a given frequency range, either modulated or non-modulated.

Said radiofrequency identifier further comprises an identification chip 10.

Said identification chip 10 is operationally connected to the said insert antenna 8, and is configured to transmit, when activated, information related to the insert assembly 1.

Additionally, said medical device handpiece 4 comprises a handpiece antenna 12.

Said ferromagnetic layer 6, dielectric layer 7, and insert tip antenna 8 form a transceiver device 11, adapted to wirelessly connect said identification chip 10 to said handpiece antenna 12 of said handpiece 4.

Advantageously, said ferromagnetic layer 6 and/or said dielectric layer 7 comprises a chip housing 13.

Said identification chip 10 is operationally connected to said insert antenna 8 in such a way to avoid protruding from at least one first side, or outer side 14, of said substantially flat profile P of said insert antenna metal element 9 of said insert antenna 8.

Furthermore, said identification chip 10 protrudes with a chip portion 16 thereof from the opposite side, relative to said outer side 14 of said substantially planar profile P, protruding from the inner side 15 of said insert antenna metal element 9 of said insert antenna 8.

Advantageously, said chip portion which protrudes 16 is accommodated in said chip housing 13, making said radiofrequency identifier particularly compact and space-saving.

Furthermore, the insertion of said chip portion which protrudes 16 into said chip housing 13 further shields said identification chip 10 making it more robust to external electromagnetic disturbances and also the assembly of said identification chip 10 and said insert antenna 8 more robust mechanically to external stresses and more thermally protected from thermal stresses, e.g., applied by sterilization autoclaves or washer-disinfectors.

According to an embodiment, said ferromagnetic layer 6, said dielectric layer 7 and said insert antenna 8 form a stack having dimensions in the range between 50 micrometers and 800 micrometers, preferably between 100 micro-meters and 600 micro-meters and adapted to be placed on top of an insert with dimensions transverse to its longitudinal extension, e.g., radial dimensions at one of its longitudinal extension axes, not exceeding 6,400 micrometers. These values refer to the thickness of the stack on a plane so they represent the increase of the radius of the insert tip 2 if, by way of example, it is cylindrical, while the radial dimensions of the insert tip are the diameter of the metal of insert tip 2 if, for example, it is cylindrical.

According to an embodiment, the thickness of the ferromagnetic layer 6, i.e., the radial dimension relative to the longitudinal extension of the insert tip 2, is in the range between 20 micrometers and 400 micrometers, preferably between 50 micrometers and 300 micrometers.

According to an embodiment, an inner insulating layer 17, e.g., double-sided adhesive, is arranged internally relative to said ferromagnetic layer 6, i.e., between said insert metal tang 5 and said ferromagnetic layer 6.

According to an embodiment, said dielectric layer 7 is a two-sided adhesive.

According to an embodiment, said insert antenna 8 is made of aluminum.

According to an embodiment, said insert antenna 8 has a flat rectangular shape and dimension of the rectangle sides of the respectively of the short side between 1 mm and 6mm, preferably between 2mm and 4mm, and of the long side between 10mm and 30mm and preferably between 12mm and 25mm, and thickness of said insert antenna 8 less than 50 micrometers.

According to an embodiment, said radiofrequency identifier 3 further comprises an outer insulation layer 18, placed externally relative to said ferromagnetic layer 6, dielectric layer 7, identification chip 10 and insert antenna 8.

According to an embodiment, said radiofrequency identifier 3 further comprises a protective layer 19, e.g., biocompatible, arranged externally relative to said ferromagnetic layer 6, dielectric layer 7, identification chip 10 and insert antenna 8.

According to an embodiment, said radiofrequency identifier 3 further comprises a protective layer 19, e.g., biocompatible, arranged externally relative to said outer insulating layer 18.

According to an embodiment, said outer insulating layer 18 is made of PVC (polyvinyl chloride), or PET polyethylene terephthalate, or polyamide, e.g., Kapton^{®}.

According to an embodiment, said protective layer 19 is biocompatible, e.g., a paint or epoxy compound with one or more components.

According to an embodiment, said ferromagnetic layer 6, said dielectric layer 7 and said insert antenna 8 form a stack.

According to an embodiment, said ferromagnetic layer 6, said dielectric layer 7 and said insert tip antenna 8 are wound about said insert metal tang 5 forming a substantially concentric structure around said central metal element 5. The term "concentric" does not mean that the structure is totally circling the central metal element nor that it must be perfectly concentric thereto, but only that it embraces the inner metal element along a portion of the periphery of the central metal element, e.g., to allow a minimum extension of said insert antenna about the periphery of the insert tip, an extension adapted for the desired transmission/reception.

According to an embodiment, such ferromagnetic layer 6 comprises ferrite. According to an embodiment, said ferromagnetic layer 6 is actually made of thin sintered ferrite with high permeability or a polymer base, mixed with magnetic powders of micrometric size dispersed throughout the material.

According to an embodiment, said ferromagnetic layer 6 said identification chip 10 is an RFID TAG chip.

According to an embodiment, said identification chip 10 has a parallelepiped-shape with base side dimensions between 50 micrometers and 1200 micrometers, preferably between 100 micrometers and 1000 micro-meters, and thickness less than 300 micrometers.

According to an embodiment, said insert assembly 1 is configured to operate in association with a medical device for dental or prophylactic or implantological and medical applications in the maxillofacial or craniofacial or neuro-spinal or orthopedic or other anatomical districts.

The present invention further relates to a medical device 20 comprising a control element 21, a medical device handpiece 4 equipped with a handpiece antenna 12 for RF transmitting-receiving, and an insert assembly 1 according to any one of the embodiments described above.

According to an embodiment, said insert assembly 1 is operatively and mechanically connected separably from said handpiece 4.

According to an embodiment, said medical device handpiece 4 comprises a handpiece distal portion 22 which ends with a distal handpiece end 30, a handpiece central portion 23, and a handpiece proximal portion 24. A transducer 25, e.g., a piezoelectric transducer, is connected to an ultrasound generator or control unit 26. Said transducer 25 is accommodated in said medical device handpiece 4 and an insert attachment tang 27 protrudes through said handpiece distal portion 22 connecting in a removable manner to said insert 2 to, when activated, put said insert 2 into resonance.

According to an embodiment, said handpiece distal portion 22 comprises said handpiece antenna 12. According to an embodiment, said handpiece antenna 12 connects to said handpiece 4 in a movable manner. According to an embodiment, said handpiece antenna 12 is contained in said handpiece 4 and connects to said handpiece 4 in a fixed manner.

According to an embodiment, said handpiece distal portion 22 comprises a handpiece antenna connection element 28.

Said handpiece antenna 12 is supported and electrically connected to said handpiece antenna connection element 28 and protrudes from said handpiece antenna connection element 28 towards said distal handpiece end 30 to at least partially overlap said insert antenna 8 when said insert tip is connected to said handpiece 4.

According to an embodiment, said handpiece antenna 12 protrudes from said handpiece antenna connection element 28 towards said distal handpiece end 30 so as not to overlap said insert antenna 8 when said insert tip is connected to said handpiece 4.

According to an embodiment, said handpiece antenna connection element 28 is electrically connected to said handpiece in a fixed or removable manner. Said handpiece antenna connection element 28 may comprise at least one LED 29.

According to an embodiment, each handpiece antenna connection element 28 does not comprise LEDs 29.

According to an embodiment, if said handpiece antenna connection element 28 comprises at least one LED 29, it may comprise a light guide element 31 comprising at least one light concentrator 32 is associated with said handpiece antenna connecting element 28 to guide the light of at least one LED 29 at the distal handpiece end 30 to illuminate the working area of said medical device 20.

According to an embodiment, said light guide element 31, said handpiece antenna connection element 28 and said handpiece antenna 12 are covered by a distal handpiece portion cap 33, e.g., made of aluminum, connected in a removable manner to said distal handpiece portion 22.

According to an embodiment, said medical device 20 connects said handpiece 4 to said ultrasound generator or control unit 21 by means of a connecting cable 34 for supplying power and fluid.

According to an embodiment, said medical device handpiece 4 receives inserts / insert tips 2 mechanically coupled to a device generating ultrasonic micro-vibrations, e.g., piezoelectric transducer 25, interchangeably, and operating at different frequencies and ranges of power and ultrasonic wave amplitude as a function of the chosen type of insert 2.

According to an embodiment of the insert assembly, the insert antenna 8 is an RF antenna adapted to work at radiofrequency, the identification chip 10 is a radiofrequency identification chip and the aforesaid frequency range comprises one or more radiofrequency ranges.

According to an embodiment of the insert assembly 1, the aforesaid transceiver device (hereinafter also referred to as "transceiver structure") formed by ferromagnetic layer 6, dielectric layer 7 and insert antenna 8 can be modeled by means of an electrical circuit in which the electrical parameters depend on the size and material of the ferromagnetic layer 6.

According to an implementation option, the aforesaid transceiver structure formed by ferromagnetic layer 6, dielectric layer 7, insert antenna 8, and identification chip 10, can be modeled by means of an electric circuit LC, in which the inductance La and capacitance Cc parameters depend on the dimensions and material of ferromagnetic layer 6, dielectric layer 7, insert antenna 8 and identification chip 10. In particular, the inductance La depends mainly on the dimensions and material of the ferromagnetic layer, the dielectric layer and the insert antenna, while the capacitance Cc depends mainly on the identification chip 10.

In this case, the frequency range over which the transceiver structure can operate depends on the aforesaid inductance La and capacitance Cc parameters.

According to an option of use of the insert assembly, the operating frequency ranges of the transceiver structure comprise frequency ranges in the UHF RFID band (between 860MHz and 960MHz): for example, 865-868MHz ETSI European technical standard, 902-928MHz FCC FHSS North American technical standard, 916.7-920.9MHz and 916.7-923.5MHz MIC LBT Japanese technical standard, 902-907.5MHz and 915-928MHz ANATEL FHSS Brazilian technical standard, 920.5-924.5MHz MII FHSS Chinese technical standard.

According to an implementation option of the insert assembly, the thickness of the ferromagnetic layer, i.e., the radial dimension relative to the insert, is in the range between 20 micrometers and 400 micrometers.

More preferably, the thickness of the ferromagnetic layer, i.e., the radial dimension relative to the insert, is comprised in the range between 50 micrometers and 300 micrometers.

According to different possible implementation options of the insert assembly, the ferromagnetic layer is actually made of thin sintered ferrite with high permeability or a polymer base, mixed with magnetic powders of micrometric size dispersed throughout the material.

According to an embodiment of the insert assembly 1, the identification chip 10 is an RFID chip (e.g., an RFID chip known in itself).

According to an embodiment of the insert tip assembly 1, the identification chip 10 is configured to store, and to transmit, when excited, through the aforesaid transceiver structure, one or more pieces of information belonging to the following assembly:
- unique and non-modifiable identification code information of the insert assembly;
- information about the manufacturer and traceability of the insert assembly, and/or one or more types of medical instruments in which the insert assembly may operate;
- information about the operating frequency ranges in which the transceiver structure of the insert assembly can operate;
- information that the insert tip screwed to the handpiece is appropriate for the type of surgery selected in the medical device;
- information about the history of modes and times of use;
- information about the integrity of the insert assembly to maximize the efficiency and effectiveness of the clinical-surgical phase towards the patient;
- information about the integrity of the insert assembly for appropriate scheduled maintenance;
- information about whether or not the insert assembly is inserted correctly, if the insert assembly is inserted in a respective handpiece;
- information about operating parameters of the insert assembly and/or error or alarm messages in the presence of anomalous operating situations.

According to an example of use, the insert assembly is configured to operate in association with a medical device for dental or prophylactic or implantological and medical applications in the maxillofacial or craniofacial or neuro-spinal or orthopedic or other anatomical districts.

A medical device according to the present invention is described here.

Such a medical device comprises a control unit, a handpiece with a handpiece antenna and an insert assembly according to any one of the embodiments described above, in which such insert assembly is operationally and mechanically connected to the aforesaid handpiece of the medical device, and wherein the insert antenna is configured to communicate wirelessly with the handpiece antenna.

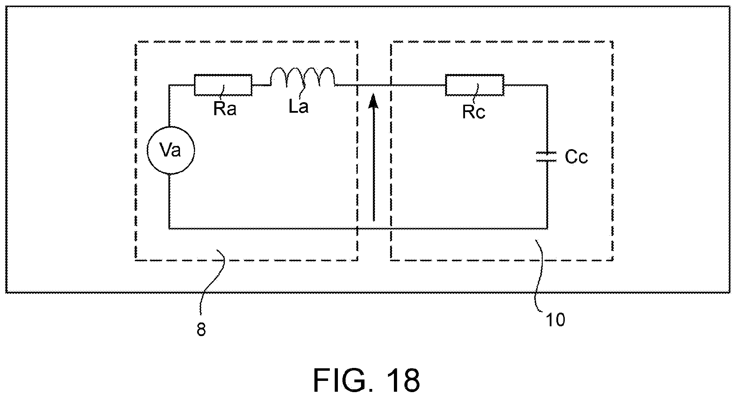

With reference to figures 18-20, further information about possible embodiments of the insert assembly will be given hereinafter.

Figure 18 shows an equivalent electrical circuit in the series representation of the assembly formed by the RFID identification chip 10 and the insert antenna 8. Ignoring the parasitic capacitance of the whole, the identification chip 10 is modeled by a capacitance Cc and a resistor Rc; the insert antenna 8 is modeled by an inductance La and a resistor Ra. Indeed, the overall electrical circuit is an LC circuit, while the resistive components take "non-idealities" into account, i.e., radiated system losses and energy losses.

Such model represents an embodiment in which the wireless transceiver structure is based on Near Field UHF (860MHz-960MHz) technique, and exploits the reactive part of the electromagnetic field, which is operating as a so-called "magnetic antenna" with closed-loop geometry preferably segmented.

From the point of view of the fields, the ferromagnetic layer has the function of isolating the wireless transceiver structure from the metal parts of the insert /insert tip body, thus preventing the creation of the reflected antagonist field caused by the stray currents induced by the variable primary magnetic field, thus avoiding the phenomena of attenuation or zeroing of the overall electromagnetic field.

In other words, the addition of the ferromagnetic layer, with a complex effective magnetic permeability, modifies the magnetic profile by influencing the mutual inductance and self-inductance. By appropriately selecting material and dimensions of the ferromagnetic layer (e.g., as in the abovementioned implementation options ) it is possible to have degrees of freedom to optimize the design of the transceiver structure, maximizing the intensity of the overall field present at the antenna, and improving communication as desired.

From a "concentrated parameters" electrical point of view, the ferromagnetic layer determines the effect of increasing the inductance of the LC system (where C is dominated by the chip). This feature, in turn, determines the additional effect of lowering the resonance frequency of the "antenna-chip" system, which has the advantage of bringing it within the limits required by the UHF band (and thus facilitating and improving the design of the transceiver structure, making it more easily suited to the desired uses).

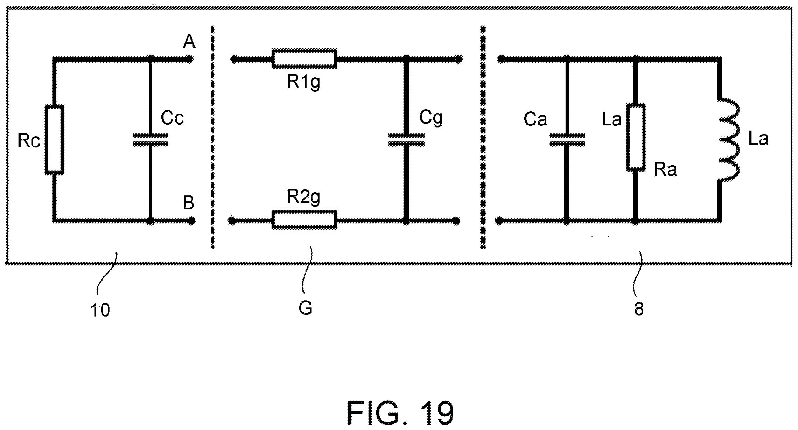

Figure 19 shows a parallel representation of another example of an equivalent electrical circuit of the assembly formed by the RFID identification chip 10 and the insert antenna 8, also including a model of the connection G. The representation includes system parasitic elements, such as antenna parasite capacitance.

In particular, in this case, the transceiver structure comprising the insert antenna 8 and the ferromagnetic layer 6 is modeled by means of an RLC circuit, while the identification chip 10 is modeled by means of an RC circuit.

From a substantial point of view, the considerations already made above with regard to figure 18 apply.

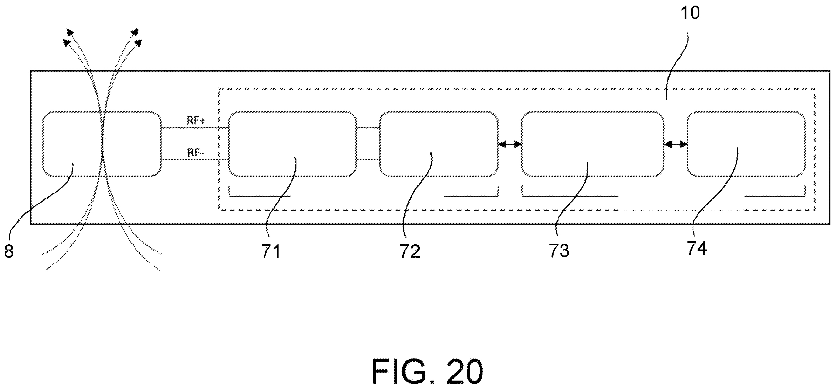

Figure 20 is a block diagram of an embodiment of the insert antenna 8 and the identification chip 10.

In particular, magnetic field lines are highlighted about the Near Field antenna 8.

The identification chip 10, in this embodiment, comprises an analog front-end (power management element 71 and modulator/demodulator element 72) and a digital control part, with a controller 73 (of a type known in itself, e.g., RFID tags) and a non-volatile memory 74.

As shown in paragraph 52 (ìx-x-xì), the non-volatile memory 74 is configured to store usage information, such as the cumulative sum of usage time and statistically estimated degree of wear, so as to provide the clinician with an updated situation even if the inserts / insert tips are used in places other than the original (clinician with several offices) but always with a suitable and compatible medical device.

According to a particular implementation option , to ensure data integrity and authenticity, the data can be stored in non-volatile memory 74 even after an encryption or password authentication process which can only be decoded by the appropriate medical device with which the insert can be associated.

A handpiece assembly for a medical device comprising a handpiece for medical device 4 is described below. Such handpiece 4 comprises a handpiece distal portion 22 adapted to receive by insertion an insert assembly 1 comprising a radiofrequency identifier 3, an insert antenna 8 and an insert 2 adapted to interact with a part of a patient's body (e.g., an insert assembly according to the embodiments described above).

The handpiece assembly for medical device further comprises a handpiece antenna 12, arranged in the handpiece distal portion 22, and radiofrequency signal supply means, configured to provide the handpiece antenna 12 with a radiofrequency signal adapted to be transmitted by the handpiece antenna 12.

The aforesaid handpiece antenna 12 is configured to wirelessly communicate with the insert antenna 8 when said insert assembly is inserted into the handpiece 4 in an insertion region R comprised in the handpiece distal portion 22.

The aforesaid handpiece antenna 12 is a single-coil loop antenna, preferably a segmented ring antenna, comprising at least two handpiece antenna segments S1, S2, electrically arranged in series, which will be named "first segment" S1 and "last segment" S2 hereinafter. A first end 41 of a first segment S1 of said at least two handpiece antenna segments is operatively connected to a first terminal 51 of said radiofrequency signal supply means, and a second end 42 of the aforesaid last segment S2 is operatively connected to a second terminal 52 of said radiofrequency signal supply means to constitute, around the aforesaid insertion region R, a coil radiating structure configured to generate in such insertion region R an electromagnetic field with a radiofrequency dependent on the aforesaid radiofrequency signal.

The single-coil loop or each of the aforesaid at least two handpiece antenna segments (S1, S2) comprise a radiant metal element (S11, S21), characterized by a respective inductance L, electrically connected in series to a capacitive element (S12, S22), having a capacitance C such as to compensate in the UHF RFID range (860MHz - 960Mhz) the effects due to the inductance L of the radiant metal element on currents circulating in the coil of the handpiece antenna 12.

According to an embodiment of the handpiece assembly, the aforesaid capacitance C of each of the capacitive elements (S12, S22) is such to further adapt the characteristic impedance of the handpiece antenna 12 to a radiofrequency generator placed in a control element 21 of the medical device 20, operatively connected to the handpiece assembly.

According to an embodiment of the aforesaid handpiece, the aforesaid handpiece antenna 12 further comprises an input impedance adaptation first electrical network 43, comprised between the aforesaid first terminal 51 and second terminal 52 of the signal supply means and the aforesaid first end of first segment 41 and second end of last segment 42 of the handpiece antenna 12; and further comprising an antenna impedance adaptation second electrical network 44, comprised in the segmented antenna ring, and electrically connected in series between two segments (S1', S2') of the aforesaid at least two handpiece antenna segments.

According to an implementation option, the aforesaid input impedance adaptation first electrical network 43 comprises an inductance-capacitance circuit LC or a capacitive circuit.

According to an implementation option, the aforesaid antenna impedance adaptation second electrical network 44 comprises a resistance-capacitance circuit RC or a capacitive circuit, configured to adapt the antenna impedance to the desired impedance value, adapted to optimize the transmission of the radiofrequency signal generated by the medical device 21 and carried along the cable 34 and the handpiece 4.

For example, such impedance value, in the UHF RFID range (860MHz - 960MHz) can be expressed with a complex number Z=R+iX having a module between 20 ohm and 80 ohm, preferably between 45 and 55 ohm.

According to an embodiment of the handpiece 4, the aforesaid radiant metal element (S11, S21) of the segmented ring antenna can be modeled by means of an equivalent electric circuit comprising an inductance L and a resistance R, wherein said inductance is in the range between 2nH and 30nH, and preferably between 4nH and 20n.H.

The aforesaid capacitive element (S12, S22), governed by the resonance relation of the system defined as LC = (ω²)⁻¹ and where ω=2πf and f the working resonance frequency, is a capacitor having a capacitance between 1pF and 12pF, preferably between 2pF and 10pF.

According to an embodiment of the handpiece 4, the aforesaid handpiece antenna 12 is configured to operate in frequency ranges in the UHF RFID band between 860MHz and 960MHz.

According to an implementation option , all antenna segments are characterized by equal capacitance C and inductance L values.

According to another implementation option , the inductance values L of the radiant metal element are different from segment to segment, and the capacitance values C of the capacitive element are different from segment to segment, depending on the inductance value L of the respective segment.

According to an embodiment, the handpiece assembly 4 further comprises a handpiece identifier 45 adapted to communicate wirelessly or by wire with the aforementioned handpiece antenna 12.

Such handpiece identifier 45 is configured to provide handpiece antenna identification information and/or information on operating conditions and/or operating frequencies of the handpiece antenna 12.

According to an embodiment of the handpiece assembly 4, the aforesaid radiofrequency signal supply means comprise a signal guide 50 and a handpiece antenna connection element 28.

The signal guide comprises, according to a preferred embodiment, a microstrip printed circuit of 50 ohm impedance configured to carry a radiofrequency supply signal from a connection cable 34, between the handpiece 4 and a control element 21 of the medical device 20, to the handpiece antenna 12.

According to another embodiment, the signal guide 50 comprises a miniaturized coaxial cable of 50ohm impedance.

The handpiece antenna connection element 28, connected to said signal guide 50 and the handpiece antenna 12, comprises said first terminal 51 and second terminal 52 of the radiofrequency signal supply means.

According to an embodiment, the aforesaid microstrip circuit is a 50ohm impedance-controlled circuit, comprising metal tracks that are less than one millimeter thick.

The aforesaid microstrip circuit can be made by means of a multilayer printed circuit based on technologies known per se.

According to a particular implementation option , the aforesaid microstrip circuit comprising metal tracks is comprised between 0.2 mm and 1.0 mm thick, preferably between 0.35 mm and 0.85 mm, and is made of Vetronite material type FR4 or Kapton - Polyamide or Rogers.

According to an embodiment of the handpiece assembly, the aforesaid coil radiating structure of the handpiece antenna has a diameter smaller than 30mm to be contained within an inner wall of the handpiece distal portion 22.

According to an embodiment of the handpiece assembly, the handpiece antenna 12 is connected in either fixed or separable manner to the handpiece distal portion 22.

According to a particular embodiment of the handpiece assembly, the handpiece antenna 12 is made from a T or Γ shaped flexible rigid multilayer printed circuit board, in which the flexible part (e.g. Kapton - polyamide) extends from the rigid part (e.g., Vetronite type FR4 or Rogers).

When the aforesaid flexible rigid multilayer printed circuit board is placed in position inside the cone of the handpiece, the upper part of the T or Γ is wound about the cone of the light guide making the edges of the upper part of the T or Γ overlap, allowing the electrical contact (i.e., forming the loop or the segmented ring), for example by means of tin or ultrasound welding.

In this case, exactly in the joining part of the vertical segment of the T or Γ with the horizontal segment, a TAG is placed, electrically connected through a decoupling capacitor. Such TAG performs the function of the aforesaid handpiece identifier and performs the function of identifying the handpiece antenna cone, e.g., for the purpose of traceability, verifying correct use relative to the geographical area (for example, according to the continent or country of destination - e.g., Europe, USA or Japan - the need arises to adapt the handpiece antenna to the local working frequency, which can be achieved with a specific set of antenna capacitors with an appropriate value, adapted to the correct working frequency) and compatibility of the insert tip relative to the clinical application.

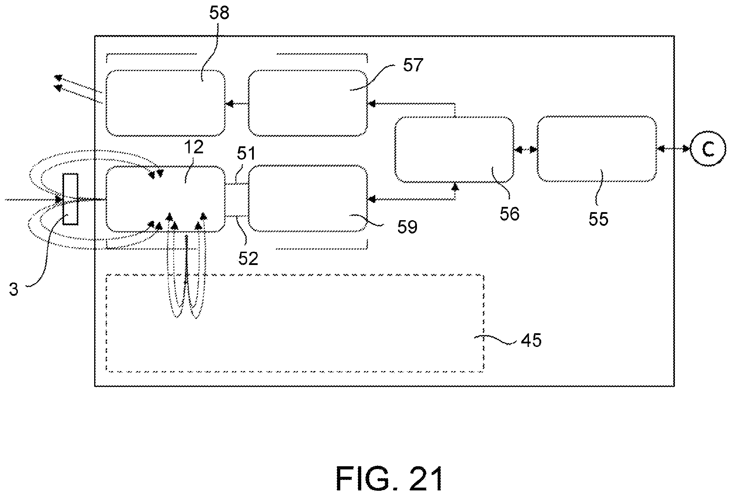

With reference to figures 21-24, further information about possible embodiments of the medical device handpiece assembly will be provided below.

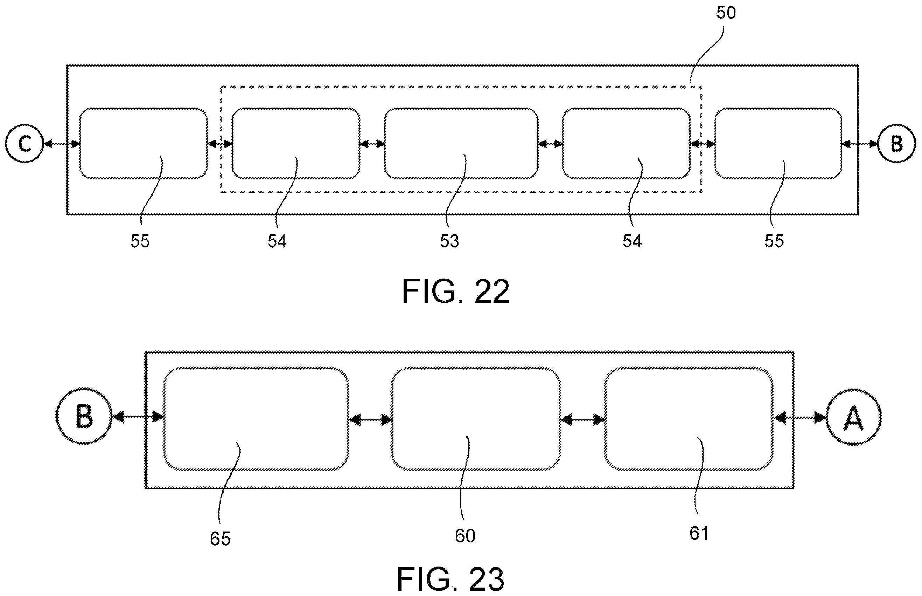

Figure 22 shows a simplified block diagram of the RF signal distribution mode in the handpiece. In such block diagram, a microstrip circuit 50 is indicated in the aforesaid embodiment comprising a multi-layer flexible circuit, which, in turn, comprises an impedance-adapted circuit on printed circuit 53 and, at each end of such circuit 53, a respective RF 54 impedance-adapted network.

The microstrip 50 circuit, in this embodiment, has the function of transmitting, with the lowest possible losses, a composite signal, including both the RF signal for the handpiece antenna and the DC signal for the LED lighting circuit of the light cone.

The microstrip or stripline circuit is an established technique in the field of impedance controlled printed circuit boards, in which a high degree of integrity of highfrequency signals is to be maintained. Additionally, this microstrip circuit has very small dimensions (in particular, thickness), thus adapting to the small inner space available in the handpiece. For example, the microstrip is designed to obtain a thickness in the range between 0.2 - 1.0mm, and preferably between 0.35 - 0.85mm.

Upstream of the microstrip circuit (reference point indicated as B in figures 22 and 23) there is a minimum RF area connector 55 with pin connections, configured to connect to a corresponding minimum RF area connector 65 with pin connections interfaced with a coaxial 60 cable.

Such coaxial cable 60 (shown in figure 23) connects the handpiece 4 to the control element 21 of the medical device and is used to transmit the RF signals between the handpiece 4 and the control element 21.

Returning to figure 22, downstream of the microstrip circuit (reference point indicated as C in figures 22 and 21) there is another minimum RF area connector 55 with pin connections, configured to connect to a corresponding further minimum RF area connector 55 with pin connections interfaced with an RF-DC decoupling network (or decoupler) 56.

Indeed, in this case, in the flexible rigid circuit, an RF-DC decoupler 56 is also obtained (shown in figure 21), which separates the RF and DC components, and routes the DC component to the LED lighting circuit 57 of the light cone, and the RF component to the handpiece antenna 12.

The aforesaid minimum area RF connectors 55 with pin connections are configured to minimize the insertion area, such as to minimize RF radiation losses at the interconnection points, and to allow adequate transmission performance even in a context where the known RF connectors for coaxial cables cannot be used due to dimensions.

Figure 21 also shows the functional blocks corresponding to the lighting circuitry 57 and the light signal guides DC 58, and the handpiece antenna 12, which is a near field antenna with RF input impedance adjustment 59.

Figure 21 also shows the functional blocks corresponding to the identifier (tag) of the handpiece 45, connected in wireless near field mode or wired mode by means of decoupling capacitor with the handpiece antenna 12.

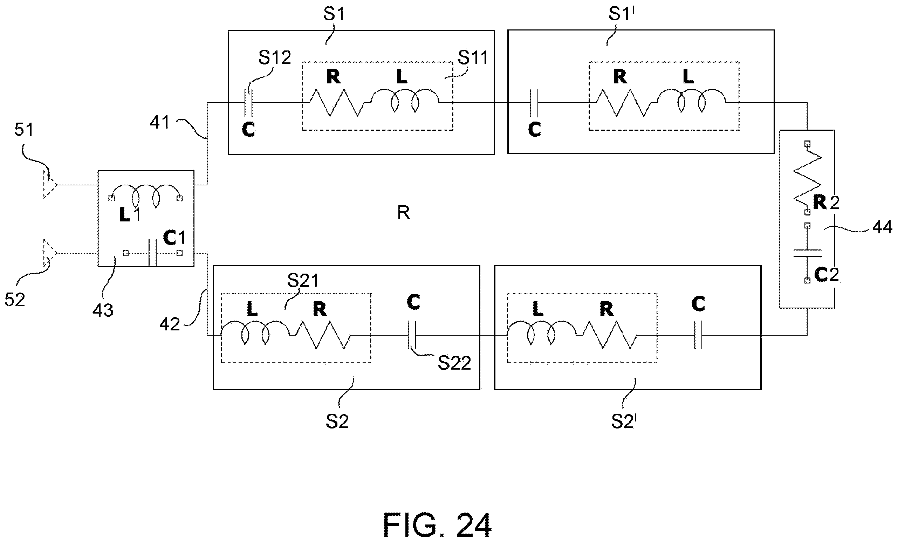

Figure 24 shows an equivalent electrical circuit of the handpiece antenna, according to an embodiment.

Hereinafter, a medical device 20 is described comprising a control element 21, a medical device handpiece 4 according to any one of embodiments described above and an insert assembly 1 comprising a radiofrequency identifier 3, an insert antenna 8 and an insert 2 adapted to interact with a part of a patient's body.

The aforesaid insert assembly 1 is operatively and mechanically connected separably from the handpiece 4 of the handpiece assembly.

The aforesaid insert antenna 8 and handpiece antenna 12 are configured to communicate with each other wirelessly in radiofrequency.

According to an embodiment, the medical device 20 is configured in such a way that, when the insert assembly 1 is connected to the handpiece 4, the insert antenna 8 is arranged in the vicinity of the handpiece antenna 4, in the aforesaid insertion region R in which the radiofrequency electromagnetic field generated by the handpiece antenna 4 is present in radiant condition.

As can be noted, the purpose of the present invention is fully achieved by the system chain (insert assembly 1 - handpiece assembly 22;4;24 - cable 34 - medical device 20 - control device 21) developed and described above, by virtue of its structural and functional features.

Indeed, by virtue of the features described in detail above, the insert assembly allows to reduce and minimize undesired phenomena which worsen the communication between the insert assembly and the remaining parts of the medical device.

In particular, from the point of view of the fields, the ferromagnetic layer has the function of isolating the wireless transceiver structure from the metal parts of the insert body, preventing the creation of the reflected antagonist field and thus avoiding the phenomena of attenuation or zeroing of the overall electromagnetic field (complained in the prior art).

The addition of the ferromagnetic layer, with a complex effective magnetic permeability, modifies the magnetic profile by influencing the mutual inductance and self-inductance. By appropriately selecting material and dimensions of the ferromagnetic layer (e.g., as in the abovementioned implementation options) it is possible to obtain degrees of freedom to optimize the design of the transceiver structure, maximizing the intensity of the overall field present at the antenna, and improving communication as desired.

Ultimately, this leads to an improvement in the patient's safety requirement, which is extremely important in the technical and application areas indicated, but in particular in the dental and medical areas defined in the document.

From a "concentrated parameters" electrical point of view, the ferromagnetic layer increases the inductance of the system LC (where C is represented by the chip). This feature, in turn, determines the further effect of lowering the resonance frequency of the "antenna-chip" system, which has the further advantage and reaches the further desired purpose of bringing it within the limits required by the UHF band, and thus facilitating and improving the design of the transceiver structure, making it more easily suited to the desired uses.

With reference to the requirements for the handpiece antenna, it is worth noting that the handpiece antenna, by virtue of the functional and structural characteristics described above, has very small dimensions (suitable for the context of use), can be easily manufactured and provides adequate transmissive performance.

Indeed, by virtue of the loop or segmented loop structure and the positioning in the distal handpiece portion, about the insertion region of the insert assembly, the handpiece antenna has a uniform emission lobe over its entire emission zone and generates a stable and sufficiently intense electromagnetic field precisely in the insertion region of the insert assembly.

The structure of the segmented ring antenna, comprising line sections connected by capacitors which compensate and/or cancel with their capacitance the inductance of the corresponding copper line segment, avoids the phase inversion of the current which circulates on the coil, thus obtaining a uniform and strong field especially in the center of the coil, in a region in which the insert antenna intended to communicate with the handpiece antenna is positioned, ultimately to allow the unique insert identifier to be read and the operating and maintenance parameters of the insert to be written or read into non-volatile memory.

The series configuration of the capacitors connecting the line sections of the segmented ring antenna structure allows an accurate tuning of the resonance frequency being able to rely on the overall capacitance achieved by a multitude of capacitors guaranteeing a fine resolution of the total capacitance value rather than a single component with coarse tolerances and nominal values imposed by the market.

Furthermore, the handpiece comprises a communication line (handpiece antenna and related RF signal supply means) which can allow for adequate impedance adaptation (essential for effective RF signal transmission and emission) even in a small structure, which does not allow the use of common impedance adapted RF transmission methods (e.g., coaxial cables and related RF connectors). Such purpose is achieved, for example, through an impedance adaptation power supply network, included in the segmented ring structure of the antenna, and by a microstrip RF signal distribution circuit with the features described above.

### LIST OF REFERENCE NUMBERS

- 1: insert assembly
- 2: insert
- 3: radiofrequency identifier
- 4: medical device handpiece
- 5: insert metal tang
- 6: ferromagnetic layer
- 7: dielectric layer
- 8: insert antenna
- 9: insert antenna metal element
- 10: identification chip
- 11: transceiver device
- 12: handpiece antenna
- 13: chip housing
- 14: outer side of said substantially flat profile of said insert antenna metal element of said insert antenna
- 15: inner side of said insert antenna metal element of said insert antenna
- 16: protruding chip portion
- 17: inner insulation layer, e.g. double-sided adhesive
- 18: outer insulation layer, e.g. PVC or PET or polyamide
- 19: biocompatible protective layer, e.g. paint or mono or multiple-component epoxy compound
- 20: medical device
- 21: control element
- 22: handpiece distal portion
- 23: handpiece central portion
- 24: handpiece proximal portion
- 25: transducer e.g. piezoelectric transducer
- 26: ultrasound generator or control unit
- 27: threaded tang for insert connection
- 28: handpiece antenna connection element
- 29: LED
- 30: handpiece distal end
- 31: light guide element
- 32: light concentrator
- 33: handpiece distal portion cap
- 34: connection cable
- 41: handpiece antenna first segment first end
- 42: handpiece antenna last segment second end
- 43: handpiece antenna input impedance adaptation first electrical network
- 44: handpiece antenna antenna impedance adaptation second electrical network
- 45: handpiece identifier
- 50: signal guide of the radiofrequency signal supply means (e.g., microstrip circuit)
- 51: first terminal of the signal supply means
- 52: second terminal of the signal supply means
- 53: impedance-adapted circuit on microstrip printed circuit board
- 54: microstrip circuit RF impedance adaptation network
- 55: minimum area RF connector with pin connections
- 56: RF-DC decoupling network (or decoupler)
- 57: LED lighting circuit
- 58: guides for the DC light signal
- 59: handpiece antenna input RF impedance adaptation network
- 60: coaxial connection cable between handpiece and control unit
- 61: 50ohm RF coaxial connector
- 65: minimum area RF connector with pin connections
- 71: identification chip power management element 71
- 72: identification chip modulator/demodulator element
- 73: identification chip controller
- 74: identification chip non-volatile memory
- 101: ultrasonic system
- 102: generator means

## Claims

1. Handpiece assembly for medical device, comprising a handpiece for medical device (4), comprising a handpiece distal portion (22) adapted to receive by insertion an insert assembly (1) comprising a radiofrequency identifier (3), an insert antenna (8) and an insert (2) adapted to interact with a part of a patient's body,
wherein said handpiece assembly for medical device further comprises a handpiece antenna (12), arranged in the handpiece distal portion (22), and radiofrequency signal supply means, configured to provide the handpiece antenna (12) with a radiofrequency signal adapted to be transmitted by the handpiece antenna (12),
said handpiece antenna (12) being configured to wirelessly communicate with the insert antenna (8), when said insert assembly is inserted into the handpiece (4) in an insertion region (R) comprised in the handpiece distal portion (22),
said handpiece antenna (12) being a loop antenna or segmented ring antenna comprising:
- at least two handpiece antenna segments (S1, S2), electrically arranged in series, wherein a first end (41) of a first segment (S1) of said at least two handpiece antenna segments is operatively connected to a first terminal (51) of said radiofrequency signal supply means, and a second end (42) of a last segment (S2) of said at least two handpiece antenna segments is operatively connected to a second terminal (52) of said radiofrequency signal supply means, so as to form, around said insertion region (R), a coil radiating structure configured to generate in said insertion region (R) an electromagnetic field with a radiofrequency dependent on said radiofrequency signal,
each of said at least two handpiece antenna segments comprising a radiant metal element (S11, S21), **characterized in that** the radiant metal element (S11, S21) has a respective inductance (L), electrically connected in series to a capacitive element (S12, S22), having a capacitance (C) such as to compensate for effects due to the inductance (L) of the radiant metal element on currents circulating in the coil of the handpiece antenna (12).

2. Handpiece assembly for medical device according to claim 1, wherein said capacitance (C) of each of said capacitive elements (S12, S22) is such as to adapt the characteristic impedance of the handpiece antenna (12) to a radio frequency generator placed in a control element (21) of the medical device (20), operatively connected to the handpiece assembly (4).

3. Handpiece assembly for medical device according to claim 1 or claim 2, wherein said handpiece antenna (12) further comprises:
- an input impedance first adaptation electrical network (43), comprised between said first terminal (51) and second terminal (52) of the signal supply means and said first end of first segment (41) and second end of last segment (42) of the handpiece antenna (12);
- an antenna impedance adaptation second electrical network (44), included in the segmented antenna ring, and electrically connected in series between two segments (S1', S2') of said at least two handpiece antenna segments.

4. Handpiece assembly for a medical device according to claim 3, wherein said input impedance adaptation first electrical network (43) comprises an inductance-capacitance circuit LC or a capacitive circuit.

5. Handpiece assembly for medical device according to claim 3, wherein said antenna impedance adaptation second electrical network (44) comprises a resistance-capacitance circuit RC or a capacitive circuit, configured to adapt the antenna impedance to a desired impedance value, adapted to optimize the transmission of the radiofrequency signal.

6. Handpiece assembly for medical device according to any one of claims 1-5, wherein:
- said radiant metal element (S11, S21) of the segmented ring antenna is modellable by means of an equivalent electric circuit comprising an inductance (L) and a resistance (R), wherein said inductance is in the range between 2nH and 30nH, and preferably between 4nH and 20nH;
- said capacitive element (S12, S22) is a capacitor having a capacity C comprised in the range between 1pF and 12pF, and preferably between 2pF and 10pF.

7. Handpiece assembly for medical device according to any one of claims 1-6, wherein the inductance values (L) of the radiant metal element are different from segment to segment, and the capacitance values (C) of the capacitive element are different from segment to segment, depending on the inductance value (L) of the respective segment,
and/or wherein the inductance values (L) of the radiant metal element are equal to each other in the different antenna segments, and the capacitance values (C) of the capacitive element are equal to each other in the different antenna segments.

8. Handpiece assembly for medical device according to any one of claims 1-7, wherein said handpiece antenna (12) is configured to operate in frequency ranges in the UHF RFID band between 860MHz and 960MHz.

9. Handpiece assembly for medical device according to any one of the preceding claims, further comprising a handpiece identifier (45), adapted to communicate wirelessly or by wire with said handpiece antenna (12), said handpiece identifier (45) being adapted to provide information for identifying the handpiece antenna (12) and/or information relating to operating conditions and/or operating frequencies of the handpiece antenna (12).

10. Handpiece assembly for medical device according to any one of the preceding claims, wherein said radiofrequency signal supply means comprise:
- a signal guide (50), comprising a micro-strip circuit configured to conduct a radiofrequency supply signal from a connection cable (34), between the handpiece (4) and a control element (21) of the medical device (20), to the handpiece antenna (12);
- a handpiece antenna connection element (28), connected to said signal guide and to the handpiece antenna (12), comprising said first terminal (51) and second terminal (52) of the radiofrequency signal supply means,
and/or
wherein said micro-strip circuit is a controlled impedance circuit, with a thickness comprised between 0.2 mm and 1.0 mm, and preferably between 0.35 mm and 0.85 mm, comprising metal tracks.

11. Handpiece assembly for medical device according to any one of the preceding claims, wherein said coil radiating structure of the handpiece antenna has a diameter smaller than 30mm so as to be contained within an inner wall of the handpiece distal portion (22).

12. Handpiece assembly for medical device according to any one of the preceding claims, wherein said handpiece antenna (12) is connected in a separable manner to said handpiece distal portion (22),
and/or wherein said handpiece antenna (12) is fixedly or separably connected to a central handpiece portion (23).

13. Handpiece assembly for medical device according to any one of the preceding claims, wherein the handpiece antenna (12) is formed from a flexible rigid multilayer T-shaped or Γ-shaped printed circuit comprising a flexible printed circuit part which extends from a rigid printed circuit part,
wherein, when said flexible rigid multilayer printed circuit is placed in position inside the distal portion (22) of the handpiece (12), the upper part of the T or Γ is wound onto a cone comprised in said distal handpiece portion (22) so that the edges of the upper part of the T or Γ overlap, and form an electrical contact, forming said loop or segmented ring of the handpiece antenna (12).

14. Medical device (20) comprising a control element (21), a handpiece assembly for medical device according to any one of claims 1-13, and an insert assembly (1) comprising a radiofrequency identifier (3), an insert antenna (8) and an insert (2) adapted to interact with a part of a patient's body,
wherein said insert assembly (1) is operatively and mechanically connected separably from said handpiece (4) of the handpiece assembly,
and wherein said insert antenna (8) and said handpiece antenna (12) are configured to communicate with each other wirelessly in radiofrequency.

15. Medical device (20) according to claim 14, configured in such a way that, when said insert assembly (1) is connected to said handpiece (4I), the insert antenna (8) is arranged in the vicinity of the handpiece antenna (12), in said insertion region (R) in which there is the radiofrequency electromagnetic field generated by the handpiece antenna (12) in radiant condition.

## Patentansprüche

1. Handstückanordnung für eine medizinische Vorrichtung, umfassend ein Handstück für eine medizinische Vorrichtung (4), welche einen distalen Handstückabschnitt (22) umfasst, welcher dazu eingerichtet ist, durch Einsetzen eine Einsatzanordnung (1) aufzunehmen, welche einen Radiofrequenzidentifikator (3), eine Einsatzantenne (8) und einen Einsatz (2) umfasst, welcher dazu eingerichtet ist, mit einem Teil eines Körpers eines Patienten zusammenzuwirken,
wobei die Handstückanordnung für eine medizinische Vorrichtung ferner eine Handstückantenne (12), welche in dem distalen Handstückabschnitt (22) angeordnet ist, und Radiofrequenzsignalzufuhrmittel umfasst, welche dazu eingerichtet sind, der Handstückantenne (12) ein Radiofrequenzsignal bereitzustellen, welches dazu eingerichtet ist, durch die Handstückantenne (12) übertragen zu werden,
wobei die Handstückantenne (12) dazu eingerichtet ist, drahtlos mit der Einsatzantenne (8) zu kommunizieren, wenn die Einsatzanordnung in einem Einsatzbereich (R), welcher in dem distalen Handstückabschnitt (22) umfasst ist, in das Handstück (4) eingesetzt ist,
wobei die Handstückantenne (12) eine Schleifenantenne oder eine segmentierte Ringantenne ist, welche umfasst:
- wenigstens zwei Handstückantennensegmente (S1, S2), welche elektrisch in Reihe angeordnet sind, wobei ein erstes Ende (41) eines ersten Segments (S1) der wenigstens zwei Handstückantennensegmente betriebsmäßig mit einem ersten Anschluss (51) der Radiofrequenzsignalzufuhrmittel verbunden ist und ein zweites Ende (42) eines letzten Segments (S2) der wenigstens zwei Handstückantennensegmente betriebsmäßig mit einem zweiten Anschluss (52) der Radiofrequenzsignalzufuhrmittel verbunden ist, um herum um den Einsatzbereich (R) eine strahlende Spulenstruktur zu bilden, welche dazu eingerichtet ist, in dem Einsatzbereich (R) ein elektromagnetisches Feld mit einer Radiofrequenz zu erzeugen, welche von dem Radiofrequenzsignal abhängig ist,
wobei jedes der wenigstens zwei Handstückantennensegmente ein strahlendes Metallelement (S11, S21) umfasst, **dadurch gekennzeichnet, dass** das strahlende Metallelement (S11, S21) eine jeweilige Induktivität (L) aufweist, welche elektrisch in Reihe mit einem kapazitiven Element (S12, S22) verbunden ist, welches eine derartige Kapazität (C) aufweist, dass Effekte aufgrund der Induktivität (L) des strahlenden Metallelements auf Ströme kompensiert werden, welche in der Spule der Handstückantenne (12) zirkulieren.

2. Handstückanordnung für eine medizinische Vorrichtung nach Anspruch 1, wobei die Kapazität (C) jedes der kapazitiven Elemente (S12, S22) derart ist, dass die charakteristische Impedanz der Handstückantenne (12) an eine Radiofrequenzerzeugungseinrichtung angepasst ist, welche in einem Steuerelement (21) der medizinischen Vorrichtung (20) platziert ist, welches betriebsmäßig mit der Handstückanordnung (4) verbunden ist.

3. Handstückanordnung für eine medizinische Vorrichtung nach Anspruch 1 oder Anspruch 2, wobei die Handstückantenne (12) ferner umfasst:
- ein erstes elektrisches Netzwerk (43) zur Anpassung einer Eingangsimpedanz, welches zwischen dem ersten Anschluss (51) und dem zweiten Anschluss (52) der Signalzufuhrmittel und dem ersten Ende des ersten Segments (41) und dem zweiten Ende des letzten Segments (42) der Handstückantenne (12) umfasst ist;
- ein zweites elektrisches Netzwerk (44) zur Anpassung einer Antennenimpedanz, welches in dem segmentierten Antennenring umfasst ist und elektrisch in Reihe zwischen zwei Segmente (S1', S2') der wenigstens zwei Handstückantennensegmente geschaltet ist.

4. Handstückanordnung für eine medizinische Vorrichtung nach Anspruch 3, wobei das erste elektrische Netzwerk (43) zur Anpassung einer Eingangsimpedanz eine Induktivität-Kapazität-Schaltung LC oder eine kapazitive Schaltung umfasst.

5. Handstückanordnung für eine medizinische Vorrichtung nach Anspruch 3, wobei das zweite elektrische Netzwerk (44) zur Anpassung einer Antennenimpedanz eine Widerstand-Kapazität-Schaltung RC oder eine kapazitive Schaltung umfasst, welche dazu eingerichtet ist, die Antennenimpedanz an einen gewünschten Impedanzwert anzupassen, welcher dazu eingerichtet ist, die Übertragung des Radiofrequenzsignals zu optimieren.

6. Handstückanordnung für eine medizinische Vorrichtung nach einem der Ansprüche 1-5, wobei:
- das strahlende Metallelement (S11, S21) der segmentierten Ringantenne mittels einer äquivalenten elektrischen Schaltung modellierbar ist, welche eine Induktivität (L) und einen Widerstand (R) umfasst, wobei die Induktivität in dem Bereich zwischen 2 nH und 30 nH, und vorzugsweise zwischen 4 nH und 20 nH, liegt;
- das kapazitive Element (S12, S22) ein Kondensator ist, welcher eine Kapazität C aufweist, welche in dem Bereich zwischen 1 pF und 12 pF, und vorzugsweise zwischen 2 pF und 10 pF, umfasst ist.

7. Handstückanordnung für eine medizinische Vorrichtung nach einem der Ansprüche 1-6, wobei die Induktivitätswerte (L) des strahlenden Metallelements von Segment zu Segment verschieden sind und die Kapazitätswerte (C) des kapazitiven Elements abhängig von dem Induktivitätswert (L) des jeweiligen Segments von Segment zu Segment verschieden sind,
und/oder wobei die Induktivitätswerte (L) des strahlenden Metallelements in den verschiedenen Antennensegmenten zueinander gleich sind und die Kapazitätswerte (C) des kapazitiven Elements in den verschiedenen Antennensegmenten zueinander gleich sind.

8. Handstückanordnung für eine medizinische Vorrichtung nach einem der Ansprüche 1-7, wobei die Handstückantenne (12) dazu eingerichtet ist, in Frequenzbereichen in dem UHF-RFID-Band zwischen 860 MHz und 960 MHz zu arbeiten.

9. Handstückanordnung für eine medizinische Vorrichtung nach einem der vorhergehenden Ansprüche, ferner umfassend einen Handstückidentifikator (45), welcher dazu eingerichtet ist, drahtlos oder drahtgebunden mit der Handstückantenne (12) zu kommunizieren, wobei der Handstückidentifikator (45) dazu eingerichtet ist, Informationen zum Identifizieren der Handstückantenne (12) und/oder Informationen in Bezug auf Betriebszustände und/oder Betriebsfrequenzen der Handstückantenne (12) bereitzustellen.

10. Handstückanordnung für eine medizinische Vorrichtung nach einem der vorhergehenden Ansprüche, wobei die Radiofrequenzsignalzufuhrmittel umfassen:
- eine Signalführung (50), welche eine Mikrostreifenschaltung umfasst, die dazu eingerichtet ist, ein Radiofrequenzzufuhrsignal von einem Verbindungskabel (34) zwischen dem Handstück (4) und einem Steuerelement (21) der medizinischen Vorrichtung (20) zu der Handstückantenne (12) zu leiten;
- ein Handstückantennenverbindungselement (28), welches mit der Signalführung und der Handstückantenne (12) verbunden ist und den ersten Anschluss (51) und den zweiten Anschluss (52) der Radiofrequenzsignalzufuhrmittel umfasst,
und/oder
wobei die Mikrostreifenschaltung eine gesteuerte Impedanzschaltung mit einer Dicke ist, welche zwischen 0,2 mm und 1,0 mm, und vorzugsweise zwischen 0,35 mm und 0,85 mm, umfasst ist, welche Metallspuren umfasst.

11. Handstückanordnung für eine medizinische Vorrichtung nach einem der vorhergehenden Ansprüche, wobei die strahlende Spulenstruktur der Handstückantenne einen Durchmesser aufweist, welcher kleiner als 30 mm ist, um innerhalb einer inneren Wand des distalen Handstückabschnitts (22) enthalten zu sein.

12. Handstückanordnung für eine medizinische Vorrichtung nach einem der vorhergehenden Ansprüche, wobei die Handstückantenne (12) in einer trennbaren Weise mit dem distalen Handstückabschnitt (22) verbunden ist,
und/oder wobei die Handstückantenne (12) fixiert oder trennbar mit einem zentralen Handstückabschnitt (23) verbunden ist.

13. Handstückanordnung für eine medizinische Vorrichtung nach einem der vorhergehenden Ansprüche, wobei die Handstückantenne (12) aus einer flexiblen, starren, mehrschichtigen, T-förmigen oder Γ-förmigen gedruckten Schaltung gebildet ist, welche einen flexiblen gedruckten Schaltungsteil umfasst, welcher sich aus einem starren gedruckten Schaltungsteil erstreckt,
wobei, wenn die flexible, starre, mehrschichtige gedruckte Schaltung in Position innerhalb des distalen Abschnitts (22) des Handstücks (12) platziert ist, der obere Teil des T oder Γ auf einen Konus gewickelt ist, welcher in dem distalen Handstückabschnitt (22) umfasst ist, so dass die Ränder des oberen Teils des T oder Γ überlappen und einen elektrischen Kontakt bilden, wodurch die Schleife oder der segmentierte Ring der Handstückantenne (12) gebildet wird.

14. Medizinische Vorrichtung (20), umfassend ein Steuerelement (21), eine Handstückanordnung für eine medizinische Vorrichtung nach einem der Ansprüche 1-13 und eine Einsatzanordnung (1), welche einen Radiofrequenzidentifikator (3), eine Einsatzantenne (8) und einen Einsatz (2) umfasst, welcher dazu eingerichtet ist, mit einem Teil eines Körpers eines Patienten zusammenzuwirken,
wobei die Einsatzanordnung (1) betriebsmäßig und mechanisch trennbar von dem Handstück (4) der Handstückanordnung verbunden ist
und wobei die Einsatzantenne (8) und die Handstückantenne (12) dazu eingerichtet sind, drahtlos in Radiofrequenz miteinander zu kommunizieren.

15. Medizinische Vorrichtung (20) nach Anspruch 14, welche in einer derartigen Weise eingerichtet ist, dass, wenn die Einsatzanordnung (1) mit dem Handstück (4I) verbunden ist, die Einsatzantenne (8) in der Nähe der Handstückantenne (12) in dem Einsatzbereich (R) angeordnet ist, in welchem das durch die Handstückantenne (12) in einem strahlenden Zustand erzeugte elektromagnetische Radiofrequenzfeld vorhanden ist.

## Revendications

1. Ensemble pièce à main destiné à un dispositif médical, comprenant une pièce à main destiné à un dispositif médical (4), comprenant une partie distale de pièce à main (22) adaptée pour recevoir par insertion un ensemble insert (1) comprenant un identifiant radiofréquence (3), une antenne d'insert (8) et un insert (2) adapté pour interagir avec une partie du corps d'un patient,
dans lequel ledit ensemble pièce à main destiné à un dispositif médical comprend en outre une antenne de pièce à main (12), agencée dans la partie distale de pièce à main (22), et des moyens d'alimentation en signal radiofréquence, configurés pour fournir à l'antenne de pièce à main (12) un signal radiofréquence adapté pour être transmis par l'antenne de pièce à main (12),
ladite antenne de pièce à main (12) étant configurée pour communiquer sans fil avec l'antenne d'insert (8), lorsque ledit ensemble insert est inséré dans la pièce à main (4) dans une région d'insertion (R) comprise dans la partie distale de pièce à main (22),
ladite antenne de pièce à main (12) étant une antenne cadre ou une antenne à anneau segmenté comprenant :
- au moins deux segments d'antenne de pièce à main (S1, S2), agencés électriquement en série, dans lesquels une première extrémité (41) d'un premier segment (S1) desdits au moins deux segments d'antenne de pièce à main est fonctionnellement connectée à une première borne (51) desdits moyens d'alimentation en signal radiofréquence, et une deuxième extrémité (42) d'un dernier segment (S2) desdits au moins deux segments d'antenne de pièce à main est fonctionnellement connectée à une deuxième borne (52) desdits moyens d'alimentation en signal radiofréquence, de manière à former, autour de ladite région d'insertion (R), une structure rayonnante en bobine configurée pour générer dans ladite région d'insertion (R) un champ électromagnétique avec une radiofréquence dépendant dudit signal radiofréquence,
chacun desdits au moins deux segments d'antenne de pièce à main comprenant un élément métallique rayonnant (S 11, S21), **caractérisé en ce que** l'élément métallique rayonnant (S11, S21) a une inductance respective (L), connectée électriquement en série à un élément capacitif (S12, S22), ayant une capacité (C) telle qu'elle compense des effets dus à l'inductance (L) de l'élément métallique rayonnant sur des courants circulant dans la bobine de l'antenne de pièce à main (12).

2. Ensemble pièce à main destiné à un dispositif médical selon la revendication 1, dans lequel ladite capacité (C) de chacun desdits éléments capacitifs (S 12, S22) est telle qu'elle adapte l'impédance caractéristique de l'antenne de pièce à main (12) à un générateur de radiofréquence placé dans un élément de commande (21) du dispositif médical (20), fonctionnellement connecté à l'ensemble pièce à main (4).

3. Ensemble pièce à main destiné à un dispositif médical selon la revendication 1 ou la revendication 2, dans lequel ladite antenne de pièce à main (12) comprend en outre :
- un premier réseau électrique d'adaptation d'impédance d'entrée (43), compris entre lesdites première borne (51) et deuxième borne (52) des moyens d'alimentation en signal et lesdites première extrémité du premier segment (41) et deuxième extrémité du dernier segment (42) de l'antenne de pièce à main (12) ;
- un deuxième réseau électrique d'adaptation d'impédance d'antenne (44), inclus dans l'anneau d'antenne segmenté, et connecté électriquement en série entre deux segments (S1', S2') desdits au moins deux segments d'antenne de pièce à main.

4. Ensemble pièce à main destiné à un dispositif médical selon la revendication 3, dans lequel ledit premier réseau électrique d'adaptation d'impédance d'entrée (43) comprend un circuit inductance-capacité LC ou un circuit capacitif.

5. Ensemble pièce à main destiné à un dispositif médical selon la revendication 3, dans lequel ledit deuxième réseau électrique d'adaptation d'impédance d'antenne (44) comprend un circuit résistance-capacité RC ou un circuit capacitif, configuré pour adapter l'impédance d'antenne à une valeur d'impédance souhaitée, adapté pour optimiser la transmission du signal radiofréquence.

6. Ensemble pièce à main destiné à un dispositif médical selon l'une quelconque des revendications 1 à 5, dans lequel :
- ledit élément métallique rayonnant (S11, S21) de l'antenne à anneau segmenté est modélisable au moyen d'un circuit électrique équivalent comprenant une inductance (L) et une résistance (R), ladite inductance étant dans la plage comprise entre 2 nH et 30 nH, et de préférence entre 4 nH et 20 nH ;
- ledit élément capacitif (S 12, S22) est un condensateur ayant une capacité C comprise dans la plage entre 1 pF et 12 pF, et de préférence entre 2 pF et 10 pF.

7. Ensemble pièce à main destiné à un dispositif médical selon l'une quelconque des revendications 1 à 6, dans lequel les valeurs d'inductance (L) de l'élément métallique rayonnant sont différentes d'un segment à l'autre, et les valeurs de capacité (C) de l'élément capacitif sont différentes d'un segment à l'autre, en fonction de la valeur d'inductance (L) du segment respectif,
et/ou dans lequel les valeurs d'inductance (L) de l'élément métallique rayonnant sont égales les unes aux autres dans les différents segments d'antenne, et les valeurs de capacité (C) de l'élément capacitif sont égales les unes aux autres dans les différents segments d'antenne.

8. Ensemble pièce à main destiné à un dispositif médical selon l'une quelconque des revendications 1 à 7, dans lequel ladite antenne de pièce à main (12) est configurée pour fonctionner dans des plages de fréquence dans la bande UHF RFID entre 860 MHz et 960 MHz.

9. Ensemble pièce à main destiné à un dispositif médical selon l'une quelconque des revendications précédentes, comprenant en outre un identifiant de pièce à main (45), adapté pour communiquer sans fil ou par fil avec ladite antenne de pièce à main (12), ledit identifiant de pièce à main (45) étant adapté pour fournir des informations pour identifier l'antenne de pièce à main (12) et/ou des informations relatives à des conditions de fonctionnement et/ou fréquences de fonctionnement de l'antenne de pièce à main (12).

10. Ensemble pièce à main destiné à un dispositif médical selon l'une quelconque des revendications précédentes, dans lequel lesdits moyens d'alimentation en signal radiofréquence comprennent :
- un guide de signal (50), comprenant un circuit microruban configuré pour conduire un signal d'alimentation radiofréquence à partir d'un câble de connexion (34), entre la pièce à main (4) et un élément de commande (21) du dispositif médical (20), jusqu'à l'antenne de pièce à main (12) ;
- un élément de connexion d'antenne de pièce à main (28), connecté audit guide de signal et à l'antenne de pièce à main (12), comprenant lesdites première borne (51) et deuxième borne (52) des moyens d'alimentation en signal radiofréquence,
et/ou
dans lequel ledit circuit microruban est un circuit à impédance commandée, ayant une épaisseur comprise entre 0,2 mm et 1,0 mm, et de préférence entre 0,35 mm et 0,85 mm, comprenant des pistes métalliques.

11. Ensemble pièce à main destiné à un dispositif médical selon l'une quelconque des revendications précédentes, dans lequel ladite structure rayonnante en bobine de l'antenne de pièce à main a un diamètre inférieur à 30 mm de manière à être contenue dans une paroi interne de la partie distale de pièce à main (22).

12. Ensemble pièce à main destiné à un dispositif médical selon l'une quelconque des revendications précédentes, dans lequel ladite antenne de pièce à main (12) est connectée de manière séparable à ladite partie distale de pièce à main (22),
et/ou dans lequel ladite antenne de pièce à main (12) est connectée de manière fixe ou séparable à une partie centrale de pièce à main (23).

13. Ensemble pièce à main destiné à un dispositif médical selon l'une quelconque des revendications précédentes, dans lequel l'antenne de pièce à main (12) est formée à partir d'un circuit imprimé multicouche rigide flexible en forme de T ou de Γ comprenant une partie de circuit imprimé flexible qui s'étend à partir d'une partie de circuit imprimé rigide,
dans lequel, lorsque ledit circuit imprimé multicouche rigide flexible est mis en place à l'intérieur de la partie distale (22) de la pièce à main (12), la partie supérieure du T ou Γ est enroulée sur un cône compris dans ladite partie distale de pièce à main (22) de sorte que les bords de la partie supérieure du T ou Γ se chevauchent, et forment un contact électrique, en formant ledit cadre ou anneau segmenté de l'antenne de pièce à main (12).

14. Dispositif médical (20) comprenant un élément de commande (21), un ensemble pièce à main destiné à un dispositif médical selon l'une quelconque des revendications 1 à 13, et un ensemble insert (1) comprenant un identifiant radiofréquence (3), une antenne d'insert (8) et un insert (2) adapté pour interagir avec une partie du corps d'un patient,
dans lequel ledit ensemble insert (1) est connecté fonctionnellement et mécaniquement de manière séparable à ladite pièce à main (4) de l'ensemble pièce à main,
et dans lequel ladite antenne d'insert (8) et ladite antenne de pièce à main (12) sont configurées pour communiquer l'une avec l'autre sans fil en radiofréquence.

15. Dispositif médical (20) selon la revendication 14, configuré de telle sorte que, lorsque ledit ensemble insert (1) est connecté à ladite pièce à main (41), l'antenne d'insert (8) est agencée au voisinage de l'antenne de pièce à main (12), dans ladite région d'insertion (R) dans laquelle se trouve le champ électromagnétique radiofréquence généré par l'antenne de pièce à main (12) dans un état rayonnant.
